# EUROPEAN PATENT SPECIFICATION

(11) **EP 4 032 508 B1**
(45) Date of publication and mention of the grant of the patent: **26.03.2025**
(21) Application number: 20865343.6
(22) Date of filing: 09.09.2020
(51) Int. Cl.: A61F 2/64, A61F 2/66, A61F 2/68, A61F 2/70

(54) **ASSIST DEVICE AND ARTIFICIAL LIMB**
ASSISTENZVORRICHTUNG UND KÜNSTLICHES GLIEDMASS
DISPOSITIF D'AIDE ET MEMBRE ARTIFICIEL

(30) Priority: 17.09.2019 JP 2019168899
(43) Date of publication of application: 27.07.2022
(73) Proprietor: Bionicm Inc., Tokyo, 113-8656 (JP)
(72) Inventor: SUN, Xiaojun, Tokyo 113-8656 (JP); HANZAWA, Masaki, Tokyo 113-8656 (JP); TAKIJO, Kota, Tokyo 113-8656 (JP)
(74) Representative: TBK
(86) International application number: PCT/JP2020/034021
(87) International publication number: WO 2021/054210

(56) References cited:
- EP-A1- 3 766 461
- WO-A1-2018/087997
- WO-A1-2018/087997
- WO-A1-2019/177022
- CN-A- 107 485 471
- US-A1- 2014 046 455
- US-A1- 2014 296 997
- US-A1- 2014 296 997

## Description

### TECHNICAL FIELD

The present invention relates to an assist device that assists in the movement of a joint, and to an artificial limb comprising the assist device.

### BACKGROUND ART

In general, an artificial limb comprises a socket fixed to an amputation surface of a foot, a knee joint connected to a lower end of the socket, and a foot portion connected to a lower end of the knee joint and serving as a grounding portion (see, for example, document WO 2018/087997 A1). Since such an artificial limb is to be worn on the body, it is extremely important to reduce the weight and the size of the artificial limb so as not to increase the burden on the body or to make the artificial limb itself bulky.

Post published document EP 3 766 461 A1 discloses an assistance device where a crank mechanism performing a transfer from a linear motion to a rotational motion is described. Moreover, an arrangement of a motor driven ball screw and a nut interposed between two elastic members is disclosed.

Document US 2014/046455 A1 is considered the closest prior art, and discloses an artificial knee. In this artificial knee, rotation of the knee joint is effected by means of two steel cables.

### SUMMARY OF THE INVENTION

### Problems to be Resolved by the Invention

However, the conventional passive artificial limb is often simple in structure and rod-like, and has limited functions. In addition, the conventional passive artificial limb is unable to copy the curve of a human leg or the shape of a calf; therefore, there is a problem that the conventional artificial limb has a poor design. Particularly, in the case of a powered artificial limb that requires a battery or electrical components including a motor and a circuit board, the entire artificial limb tends to be heavy because of the large number of components. Therefore, it is extremely important to reduce weight and size. In addition, the design of an artificial limb that copies the curve of a human leg is important.

The present invention was made in consideration of the above-mentioned problems, and has the object to provide an assist device and an artificial limb which can achieve weight reduction and miniaturization and can improve the design quality.

The object of the invention is achieved by an assist device according to claim 1. Advantageous embodiments are performed according to the dependent claims.

### MEANS OF SOLVING THE PROBLEMS

(1) The present invention is an assist device for assisting the operation of a joint and an assist device comprising a ball screw that converts a rotational motion of a screw shaft rotated by a motor into a linear motion of a nut; a linear motion member that is provided coaxially with the screw shaft and moves along the screw shaft with the nut; an elastic member provided coaxially with the screw shaft so as to be interposed between the nut and the linear motion member; and a crank mechanism that converts the linear motion of the linear motion member into a rotational motion.

According to the present invention, since the elastic member is provided coaxially with the screw shaft, the space for arranging parts can be made smaller and the entire assist device can be made thinner, which makes it possible to achieve weight reduction and miniaturization. In addition, according to the present invention, by achieving weight reduction and miniaturization, exterior parts having a design feature can be arranged, and thus the design feature can be improved.

Also, according to the present invention, since an elastic member is interposed between the nut and the linear motion member, the elastic member absorbs manufacturing errors, and mainly during the operation of the joint, plays a larger role in absorbing external shocks. In addition, when a lateral force is applied to the linear motion member from the crank mechanism, the nut must move up and down; however, according to the present invention, since the elastic member is interposed between the nut and the linear motion member, the elastic member absorbs the lateral force, thereby realizing smooth movement of the linear motion member. As a result, unnecessary force to the nut can be avoided, which can lead to avoidance of locking of the screw shaft and the nut and to reduction of the load on the nut.

(2) The present invention is also the assist device as described in (1) comprising a speed reduction mechanism for reducing the rotation speed of the motor and transmitting the power of the motor to the ball screw, and the speed reduction mechanism has a small gear fixed to a drive shaft of the motor and a large gear, which is fixed to the screw shaft, has a larger diameter than the small gear, and interlocks with the small gear via another gear or interlocks directly engaged with the small gear.

According to the present invention, since the speed reduction mechanism is provided, the torque of the motor can be increased by the speed reduction mechanism and transmitted to the ball screw. Therefore, since the necessary torque can be obtained with a low output motor, the motor can be made smaller, and thus weight reduction and miniaturization can be achieved.

Also, according to the present invention, since the small gear and the large gear interlock indirectly or directly by meshing, the power can be transmitted reliably and energy loss is small. As a result, the necessary torque can be obtained with a low output motor, so that the motor can be made smaller, and thus weight reduction and miniaturization can be achieved. In addition, as in the present invention, when gears are employed, high back-drivability can be obtained because the friction between the gears is small. Thus, the present invention, by obtaining high back-drivability, when the battery runs out, as a passive artificial limb, enables the user to walk continuously by extending and flexing the knee by inertia.

(3) The present invention is also the assist device as described in any of (1) to (2) above, which has a first elastic member provided below the nut and a second elastic member provided above the nut as the elastic member, and the linear motion member is a cylinder incorporating the nut, the first elastic member, and the second elastic member.

According to the present invention, without any relation to the operation of the motor, when a joint connected to the crank mechanism and a socket connected to a socket connection part of the joint operate, either the first elastic member or the second elastic member contracts, so that the external shock or load transmitted through the crank mechanism can be accurately absorbed, and the motor and the speed reduction mechanism, etc. can be protected. Also, according to the present invention, the restoring force generated at the time of the contracted first elastic member or the contracted second elastic member being restored supports the rotational operation of the joint. Therefore, since the necessary power can be obtained with a low output motor, the motor can be made smaller, and thus weight reduction and miniaturization can be achieved.

(4) The present invention is also the assist device as described in any of (1) to (3) above, comprising a framework supporting the screw shaft and a cover detachably attached to the framework.

According to the present invention, by installing a cover with a design feature corresponding to the user or situation (e.g., a cover having an exterior structure that copies human curves), the design feature corresponding to the user or situation can be ensured.

(5) The present invention is also the assist device as described in (4) above, in which the framework includes a lower support member rotatably supporting the lower part of the screw shaft, an upper support member rotatably supporting the upper part of the screw shaft, and a first pillar provided in front of the screw shaft to connect the lower support member and the upper support member; and the assist device comprises a rail, which is provided along the first pillar and guides the movement of the linear motion member, and a guide block, which is fixed to the linear motion member and slides along the rail.

According to the present invention, since the framework is comprised of a pair of the upper and lower support members and a first pillar, the framework itself can be made smaller, and thus weight reduction and miniaturization can be achieved. Also, according to the present invention, since the rail is provided to guide the movement of the linear motion member, the linear motion member can be moved smoothly.

(6) The present invention is also the assist device as described in (5) above, in which the motor is provided behind the screw shaft, and the framework has a pair of left and right second pillars provided on the left and right sides of the screw shaft or the motor so as to connect the lower support member and the upper support member.

According to the present invention, since it has the first pillar and the pair of left and right second pillars as the framework, the weight of a user can be firmly supported by the pair of left and right second pillars with the first pillar.

(7) The present invention is also the assist device as described in (5) or (6) above, in which the framework is provided below the lower support member so as to face the lower support member, and has a base connected to the lower support member, and the cover is attached to the left and right sides of the upper support member and is attached below or above the base.

According to the present invention, since the cover is attached to the left and right sides of the upper support member, the cover can receive the force in the right and left directions that the cover receives when the user kneels down. Also, according to the present invention, since the cover is installed below or above the base, the cover can receive the upward and downward forces that the cover receives.

(8) The present invention is also the assist device as described in any of (4) to (7) above, which includes a first support portion provided in the framework; a second support portion that moves between a position above the first support portion and a position below the first support portion by moving in unison with the linear motion member; a third elastic member, in which one end is rotatably supported by the first support portion and the other end is rotatably supported by the second support portion.

According to the present invention, since when the knee is extended, the third elastic member assists the operation of the joint, the necessary power can be obtained with a low output motor, so that the motor can be made smaller, and thus weight reduction and miniaturization can be achieved. In addition, according to the present invention, when the battery runs out, the extension is assisted by the third elastic member in accordance with the inertia, so that the extension can be performed more easily. Therefore, in addition to being able to cope with knee extension when walking quickly, knee buckling caused by not being able to extend sufficiently can be prevented.

(9) The present invention is also an artificial limb provided with the assist device described in any of (1) to (8) above.

According to the present invention, since the assist device that realizes weight reduction and miniaturization with the improved design feature is provided, as the artificial limb equipped with the assist device, it can also achieve weight reduction and miniaturization and thus can improve the design feature.

### EFFECTS OF THE INVENTION

According to the assist device described in (1) to (8) above and the artificial limb described in (9) above of the present invention, weight reduction and miniaturization can be achieved, and the design feature can be improved.

### BRIEF DESCRIPTION OF DRAWINGS

[FIG. 1] A schematic perspective view illustrating a knee joint according to a first embodiment of the present invention.
[FIG. 2] A schematic perspective view illustrating the knee joint shown in FIG. 1 with the condition of the cover and battery block removed.
[FIG. 3] A side view illustrating the knee joint shown in FIG. 1 with the condition of the pillar, cover, and battery block removed.
[FIG. 4] A front view illustrating the knee joint shown in FIG. 1 with the condition of the pillar, cover, and battery block removed.
[FIG. 5] A cross-sectional view illustrating the knee joint viewed in the arrow V-V direction shown in FIG. 4.
[FIG. 6] A cross-sectional view illustrating a cross-sectional surface of the knee joint shown in FIG. 1, which is cut near the bottom of the base and viewed from below.
[FIG. 7] A bottom view illustrating the knee joint shown in FIG. 1.
[FIG. 8] A schematic perspective view illustrating a foot joint according to a second embodiment of the present invention.
[FIG. 9] A schematic perspective view illustrating the foot joint shown in FIG. 8 with the condition of a cover removed.
[FIG. 10] A side view illustrating the foot joint shown in FIG. 8 with the condition of the pillar and cover removed.
[FIG. 11] A front view illustrating the foot joint shown in FIG. 8 with the condition of the pillar and cover removed.
[FIG. 12] A cross-sectional view illustrating the foot joint viewed in the arrow XII-XII direction shown in FIG. 11.
[FIG. 13] A side view illustrating a variation of the knee joint shown in FIG. 1 with a third elastic member added, and the condition of the pillar, cover, and battery block removed; (A) shows the knee joint extended, (B) shows the knee joint bent halfway (about 70°), and (C) shows the knee joint fully bent.
[FIG. 14] A diagram illustrating a variation of the knee joint shown in FIG. 1 with the modified speed reduction mechanism; (A) is a cross-sectional view in the direction of the arrow XIVA-XIVA shown in (B), and (B) is a bottom view with the condition of the parts below the speed reduction mechanism removed.

### EMBODIMENTS FOR IMPLEMENTING THE INVENTION

Hereinafter, referring to the drawings, a knee joint 1 and a foot joint 2 according to embodiments of the present invention are described in detail.

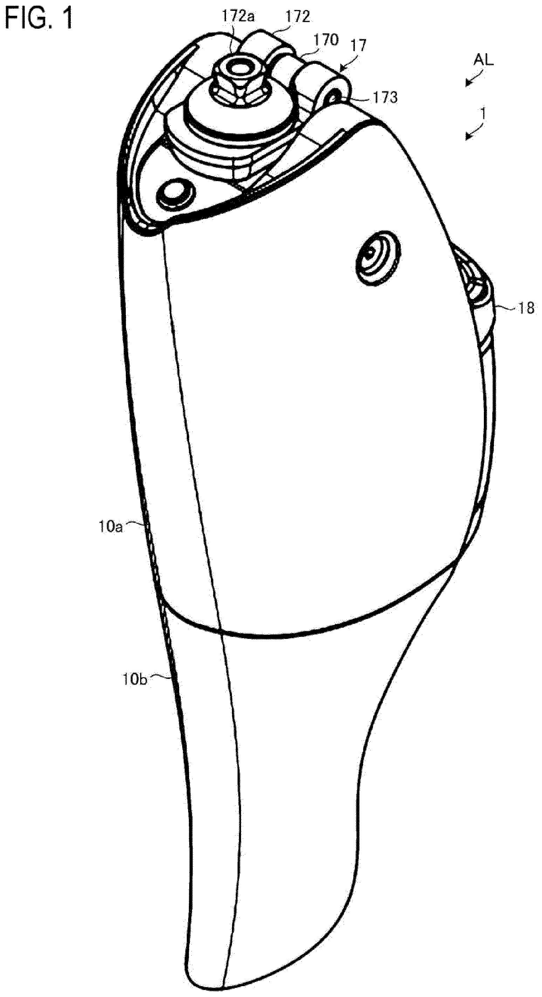

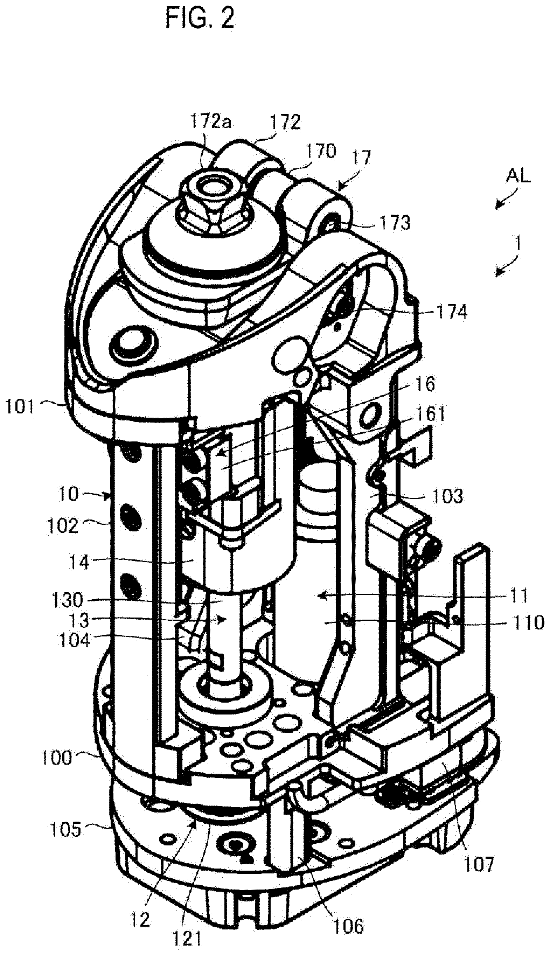

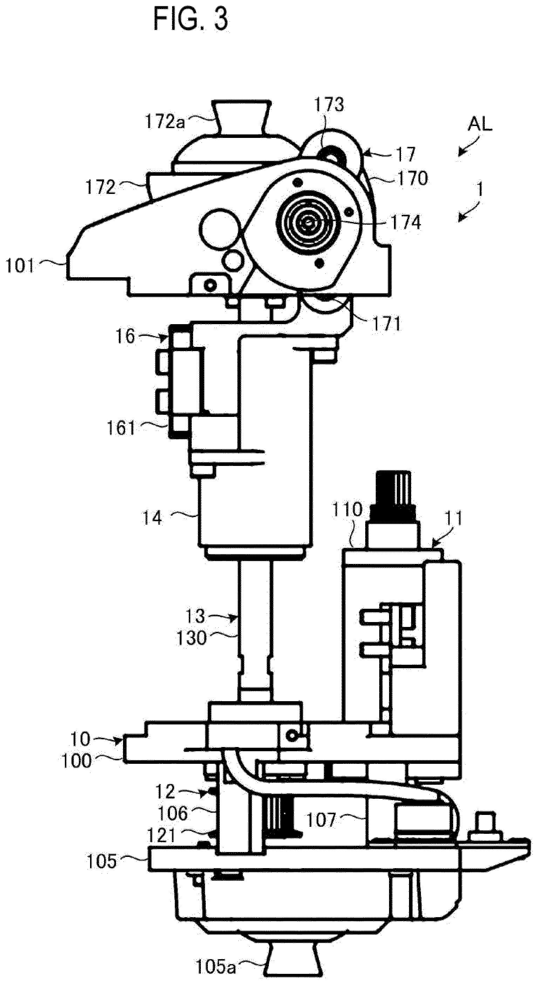

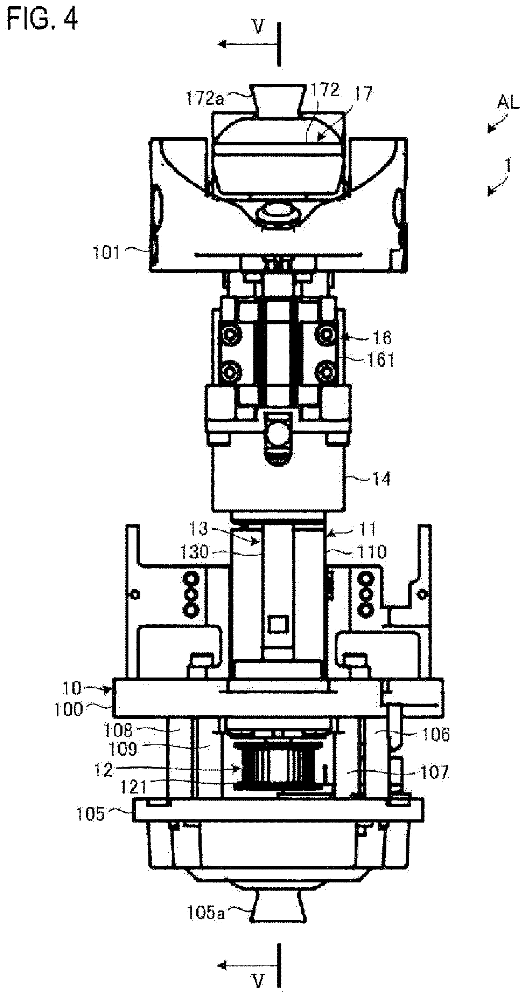

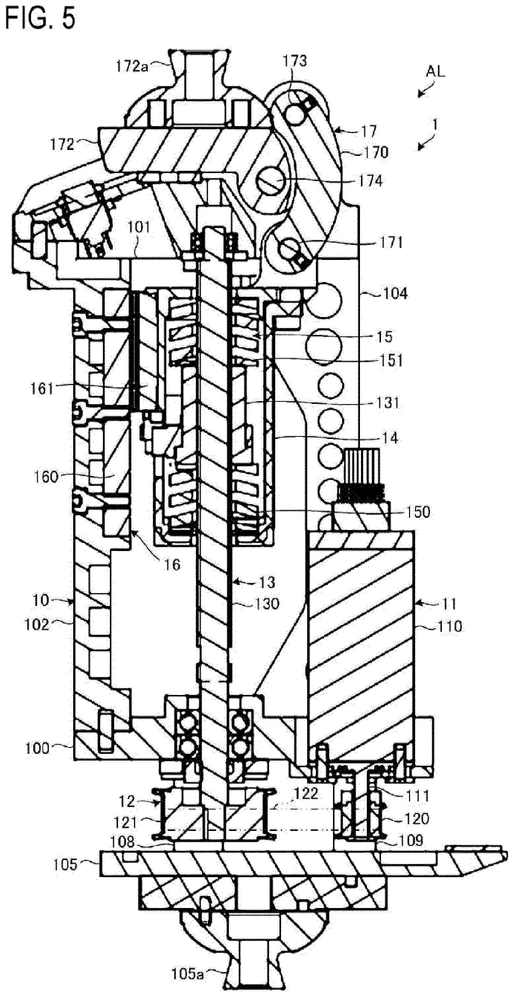

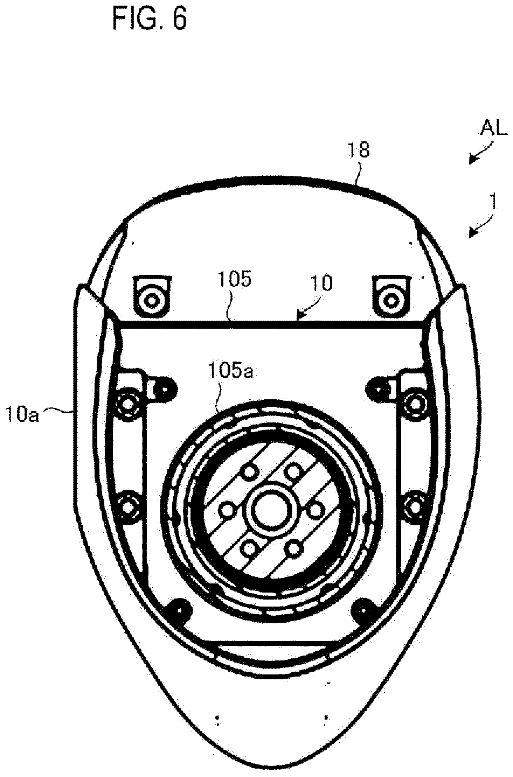

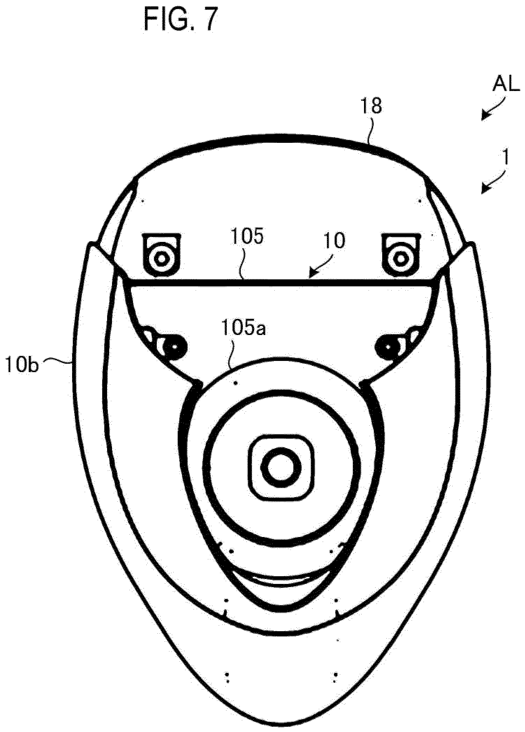

[Embodiment 1] First, using FIGS.1 to 7, the configuration of the knee joint 1 according to a first embodiment is described. FIG. 1 is a schematic perspective view illustrating the knee joint 1. FIG. 2 is a schematic perspective view illustrating the knee joint 1 with the condition of covers 10a and 10b and a battery block 18 removed. FIG. 3 is a side view illustrating the knee joint 1 with the condition of pillars 102, 103, and 104, covers 10a and 10b, and the battery block 18 removed. FIG. 4 is a front view illustrating the knee joint 1 with the condition of pillars 102, 103, and 104, covers 10a and 10b, and the battery block 18 removed. FIG. 5 is a cross-sectional view illustrating the knee joint 1 viewed in the arrow V-V direction shown in FIG. 4. FIG. 6 is a cross-sectional view illustrating a cross-sectional surface of the knee joint 1, which is cut near the lower part of a base 105 and viewed from below. FIG. 7 is a bottom view illustrating the knee joint 1.

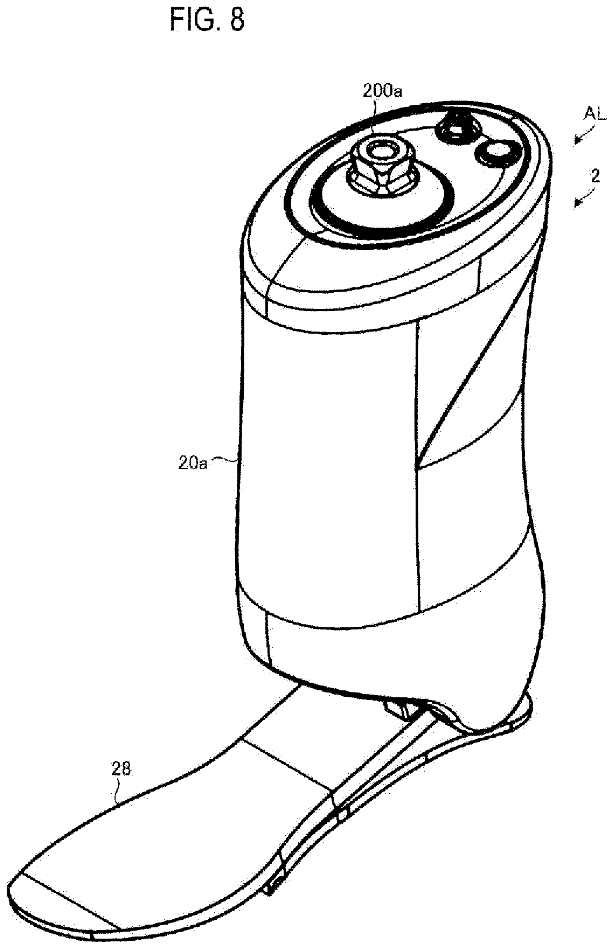

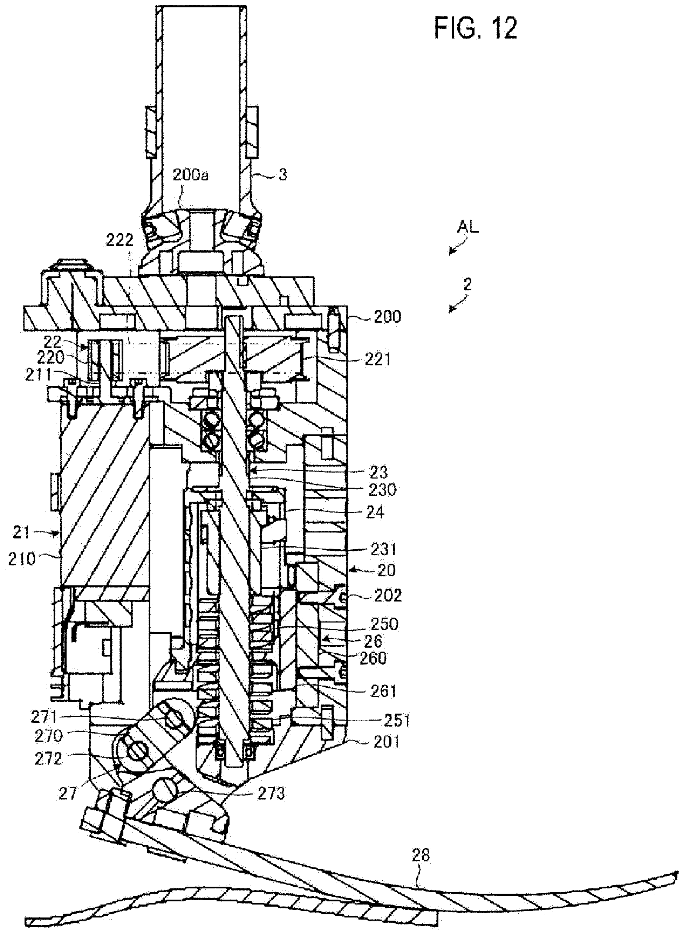

The knee joint (assist device) 1 illustrated in FIGS.1 to 7 comprises an artificial limb AL together with a socket (omitted in the figures) fixed to a cut surface of the leg, a support portion called a tube (e.g., a support portion 3 (see FIG. 12) described later), a foot joint (e.g., the foot joint 2 (see FIGS.8 to 12) described later), etc., and assists the operation of the knee joint. knee joint. The knee joint 1 is connected to the lower end of the socket (omitted in the figures) and is connected to the foot joint (e.g., the foot joint 2 (see FIGS.8 to 12) described later) via the support portion (e.g., the support portion 3 (see FIG. 12) described later).

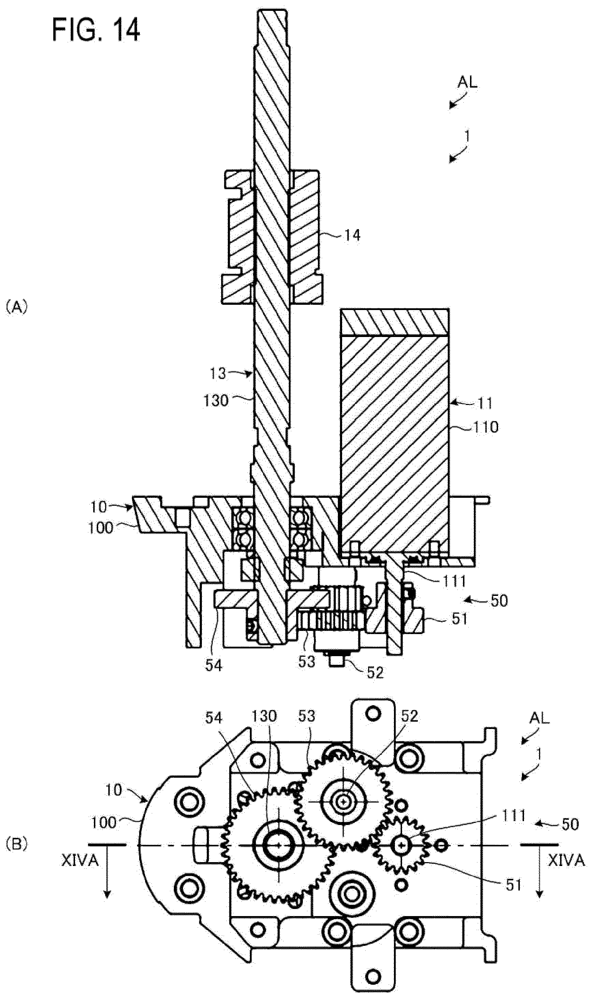

Specifically, the knee joint 1 has a framework 10, a motor 11, a speed reduction mechanism 12, a ball screw 13, a linear motion member 14, an elastic member 15, a guide member 16, a crank mechanism 17, a battery block 18, etc. It is to be noted that the described speed reduction mechanism 12 and its components merely serve for understanding of the invention, but do not form part of the invention. That is, according to the invention, a speed reduction mechanism 50 described below in context with Fig. 14 is employed.

The framework 10 forms the framework of the knee joint 1 and supports a screw shaft 130, etc. comprising the ball screw 13. The framework 10 has a pair of removable upper and lower covers 10a, 10b attached to the framework 10 itself. Specifically, the framework 10 comprises a pair of upper and lower support members 100, 101; a front first pillar 102; a pair of left and right second pillars 103, 104; a base 105; front, rear, left, and right connecting members 106, 107, 108, 109, etc.

The pair of upper and lower support members 100, 101 is positioned spaced-apart from each other so as to face each other one above the other and is connected each other by the front first pillar 102 and the pair of left and right second pillars 103, 104. The lower support member 100 fixes a motor body 110 comprising a motor 11 and rotatably supports the lower part of the screw shaft 130 comprising the ball screw 13. The upper support member 101 rotatably supports the upper part of the screw shaft 130 comprising the ball screw 13, and also relatively rotatably mounts a joint 172 comprising the crank mechanism 17 with a rotation shaft 174.

The front first pillar 102, together with a pair of left and right second pillars 103, 104, connects a pair of upper and lower support members 100, 101 to each other. The front first pillar 102 is provided in front of the screw shaft 130 along the screw shaft 130 comprising the ball screw 13 at a distance from each of the pair of left and right second pillars 103, 104. The front first pillar 102 fixes a rail 160 comprising a guide member 16 to its back side.

A pair of left and right second pillars 103, 104, together with the front first pillar 102, connects the pair of upper and lower support members 100, 101 to each other. The pair of left and right second pillars 103, 104 is spaced apart from the front first pillar 102 and from each other, and is provided on the left and right sides of the screw shaft 13 or the motor 11 along the screw shaft 130 comprising the ball screw 13.

The base 105 is provided below the lower support member 100 with a space from the lower support member 100 so as to face the lower support member 100, and is connected to the lower support member 100 by connecting members 106, 107, 108, 109 of the front, rear, left, and right connecting members. The base 105 removably attaches the battery box 18 to the rear and upper surface thereof. Also, the base 105 has a tube connection portion 105a on its lower surface. The tube connection portion 105a is for connecting a support portion (omitted in the figures), called a tube, to the knee joint 1. The tube connection portion 105a, also referred to as a pyramid connector, can be connected to a support portion 3 (see FIG. 12), referred to as a tube, using an existing method.

Front, rear, left, and right connecting members 106, 107, 108, 109 are provided between the lower support member 100 and the base 105, and connect the lower support member 100 and the base 105 so as to leave space between the lower support member 100 and the base 105.

An upper cover 10a is removably attached to the framework 10 and covers the knee joint 1 from front to both left and right sides. The upper cover 10a has an inward flange (the sign is omitted) from the lower end on both left and right sides, and is jointly fastened with the cover 10b by being screwed to the base 105 comprising the framework 10 using screw holes (omitted in the figure) provided in the flange. Also, the upper cover 10a is screwed to the left and right sides of the upper support member 101 comprising the framework 10 using screw holes (the sign is omitted) provided near the upper side on both left and right sides.

A lower cover 10b is removably attached to the framework 10 and covers the support portion 3 (see FIG. 12) from the front to both left and right sides. The lower cover 10b has an inward flange (omitted in the figure) from the upper end on both left and right sides, and is jointly fastened with the cover 10a by being screwed to the base 105 comprising the framework 10 using screw holes (omitted in the figure) provided in the flange.

The motor 11 is the power source of the knee joint 1. The motor 11 is positioned on the back and the lower side of the framework 10. That is, the motor 11 is provided behind the screw shaft 130. Specifically, the motor 11 comprises a motor body 110, a drive shaft 111, etc. The motor body 110 is fixed to the rear and upper parts of the lower support member 100 comprising the framework 10, with the drive shaft 111 facing down so that the drive shaft 111 is parallel to the screw shaft 130 comprising the ball screw 13. The drive shaft 111 penetrates through the lower support member 100 comprising the framework 10, and is arranged to protrude below the lower support member 100.

The speed reduction mechanism 12 reduces the rotational speed of the motor 11 and transmits the power to the ball screw 13. The speed reduction mechanism 12 is provided below the lower support member 100 comprising the framework 10. Specifically, the speed reduction mechanism 12 comprises a small pulley 120, a large pulley 121, an annular belt 122, etc.

The small pulley 120 has a smaller diameter than the large pulley 121. The small pulley 120 is fixed to the drive shaft 111 comprising the motor 11, and rotates by rotation of the motor 11. Also, the small pulley 120 hooks an annular belt 122 together with the large pulley 121, and the belt 122 is run by rotation of the small pulley 120 itself.

The large pulley 121 has a larger diameter than the small pulley 120. The large pulley 121 is fixed to the lower end of the screw shaft 130 comprising the ball screw 13, and also hooks the annular belt 122. Also, about the large pully 121, the annular belt 122 runs to rotate the large pulley 121 itself, and the large pulley 121 itself rotates to rotate the screw shaft 130 comprising the ball screw 13.

The annular belt 122 is hooked to the small pulley 120 and the large pulley 121. About the annular belt 122, the small pulley 120 rotates to run the annular belt 122 itself, and the annular belt 122 itself runs to rotate the large pulley 121.

The ball screw 13 converts the rotational motion of the motor 11 transmitted by the speed reduction mechanism 12 into the linear motion. The ball screw 13 is provided inside the framework 10. Specifically, the ball screw 13 is provided with the screw shaft 130, a nut 131, etc.

The screw shaft 130 is provided along each of the pillars 102, 103, 104 so as to be parallel to each of the pillars 102, 103, 104 comprising the framework 10, its lower side is rotatably supported by the lower support member 100, and its upper side is rotatably supported by the upper support member 101. The screw shaft 130 is fitted with a nut 131, and the screw shaft 130 itself rotates to move the nut 131 in the vertical direction. Also, the screw shaft 130 is coaxially provided with the linear motion member 14 and the elastic member 15.

The nut 131 is fitted to the screw shaft 130 and moves in the vertical direction as the screw shaft 130 rotates. The nut 131 is built into the linear motion member 14 with the elastic member 15, and the vertical movement of the nut 131 itself causes the linear motion member 14 to move in the vertical direction via the elastic member 15.

The linear motion member 14 is provided coaxially with the screw shaft 130 comprising the ball screw 13, and moves along the screw shaft 130 together with the nut 131 comprising the ball screw 13. The linear motion member 14 is a cylinder in which the nut 131 comprising the ball screw 13 and the elastic member 15 are built-in. Also, the linear motion member 14 has a guide block 161 comprising the guide member 16 fixed to its front side, and moves along a rail 160 comprising the guide member 16. In addition, the linear motion member 14 mounts one end of a connecting rod 170 comprising the crank mechanism 17 relatively rotatable to its upper side with a first pin 171.

The elastic member 15 is a coil spring coaxially provided with the screw shaft 130 comprising the ball screw 13 so as to be interposed between the nut 131 comprising the ball screw 13 and the linear motion member 14. Specifically, the elastic member 15 has a first elastic member 150 and a second elastic member 151.

The first elastic member 150 is positioned below the nut 131 comprising the ball screw 13. The second elastic members 151 is positioned above the nut 131 comprising the ball screw 13. These first elastic member 150 and second elastic member 151 are built into the linear motion member 14 together with the nut 131 comprising the ball screw 13.

The first elastic member 150 is pushed down by the nut 131 when the nut 131 comprising the ball screw 13 moves down, and pushes down the linear motion member 14. At this time, the second elastic member 151 is pushed down by the linear motion member 14. Also, the first elastic member 150, without any relation to the movement of the nut 131, is contracted by the external force transmitted through the linear motion member 14 when the linear motion member 14 moves up by the external force.

The second elastic member 151 is pushed up by the nut 131 when the nut 131 comprising the ball screw 13 moves up, and pushes up the linear motion member 14. At this time, the first elastic member 150 is pushed up by the linear motion member 14. The second elastic member 151, without any relation to the movement of the nut 131, is contracted by the external force transmitted through the linear motion member 14 when the linear motion member 14 is moved down by the external force.

The guide member 16 is positioned on the back of the front first pillar 102 comprising the framework 10, and guides the movement of the linear motion member 14. Specifically, the guide member 16 has the rail 160 and a guide block 161.

The rail 160 is fixed to the rear side of the front first pillar 102 comprising the framework 10 along the first pillar 102. The rail 160 engages with the guide block 161 so that the guide block 161 can slide and guides the movement of the guide block 161. The guide block 161 is fixed to the front side of the linear motion member 14 and slides along the rail 160. Therefore, the rail 160, which guides the movement of the guide block 161, guides the movement of the linear motion member 14, which is fixed to the guide block 161.

The crank mechanism 17 is positioned above the framework 10, converts the linear motion of the linear motion member 14 into the rotational motion, and transmits it to a socket (omitted in the figures) that is fixed to the cut surface of the foot. Specifically, the crank mechanism 17 has a connecting rod 170, a first pin 171, a joint 172, a second pin 173, a rotation shaft 174, etc.

The connecting rod 170 has one end mounted relatively rotatable to the upper part of the linear motion member 14 by the first pin 171, and the other end mounted relatively rotatable to the joint 172 by the second pin 173. The first pin 171 relatively rotatably attaches one end of the connecting rod 170 to the upper part of the linear motion member 14.

The joint 172 is mounted relatively rotatable to the upper support member 101 comprising the framework 10 by the rotation shaft 174 and is also mounted relatively rotatable to the connecting rod 170 by the second pin 173. The joint 172 has a socket connection portion 172a on its upper surface. The socket connection portion 172a is used to connect a socket (omitted in the figures) fixed to the cut surface of the foot to the knee joint 1. The socket connection portion 172a is rotated by the crank mechanism 17 to achieve the extension and flexion motions of the artificial limb AL. The socket connection portion 172a is also called a pyramid connector, and can be connected to a socket (omitted in the figures) that is fixed to the cut surface of the foot using an existing method.

The second pin 173 relatively rotatably attaches the connecting rod 170 to the joint 172. The rotation shaft 174 relatively rotatably attaches the joint 172 to the upper support member 101 comprising the framework 10.

The battery box 18 is the power source for the artificial limb AL including the knee joint 1. The battery box 18 is provided on the back and the lower part of the framework 10, and copies a human calf.

Next, using FIG. 5, the operation of the knee joint 1 is described.

First, the case in which the knee joint 1 drives the motor 11 is described. As the motor 11 is driven, the drive shaft 111 rotates. As the drive shaft 111 rotates, the small pulley 120 rotates in unison with the drive shaft 111. As the small pulley 120 rotates, the annular belt 122 runs. As the annular belt 122 runs, the large pulley 121 rotates. As the large pulley 121 rotates, the screw shaft 130 rotates in unison with the large pulley 121.

As the screw shaft 130 rotates, according to the rotation direction of the screw shaft 130, the nut 131 moves down or up. When the nut 131 moves in the downward direction, in unison with the nut 131, together with the first elastic member 150 and the second elastic member 151, the linear motion member 14 moves down along the rail 160. When the nut 131 moves in the upward direction, in unison with the nut 131, together with the first elastic member 150 and the second elastic member 151, the linear motion member 14 moves up along the rail 160.

When the linear motion member 14 moves in the downward direction, the connecting rod 170 moves in the downward direction. When the linear motion member 14 moves in the upward direction, the connecting rod 170 moves in the upward direction. When the connecting rod 170 moves in the downward direction, the joint 172 rotates centering around the rotation shaft 174 in the direction in which the knee joint flexes. When the connecting rod 170 moves in the upward direction, the joint 172 rotates centering around the rotation shaft 174 in the direction in which the knee joint extends.

Next, the case in which an external impact or load is applied to the knee joint 1. When an external impact or load is applied to the knee joint in the direction in which the knee joint flexes, the joint 172 rotates centering around the rotation shaft 174 in the direction in which the knee joint flexes. When an external impact is applied to the knee joint in the direction in which the knee joint extends, the joint 172 rotates centering around the rotation shaft 174 in the direction in which the knee extends. When the joint 172 rotates centering around the rotation shaft 174 in the direction in which the knee joint flexes, the connecting rod 170 moves down. When the joint 172 rotates centering around the rotation shaft 174 in the direction in which the knee joint extends, the connecting rod 170 moves up. When the connecting rod 170 moves in the downward direction, the linear motion member 14 moves in the downward direction. When the connecting rod 170 moves in the upward direction, the linear motion member 14 moves in the upward direction.

When the linear motion member 14 moves in the downward direction, the second elastic member 151 contracts. When the linear motion member 14 moves in the upward direction, the first elastic member 150 contracts. When the contracted second elastic member 151 is restored, the restoring force by the second elastic member 151 generated at that time supports the rotational movement of the joint 172. When the contracted first elastic member 150 is restored, the restoring force by the first elastic member 150 generated at that time supports the rotational movement of the joint 172.

Thus, the knee joint 1 is an assist device that assists the operation of a joint, comprising the ball screw 13 that converts a rotational motion of the screw shaft 130 rotated by the motor 11 into a linear motion of the nut 131; the linear motion member 14 that is provided coaxially with the screw shaft 130, and moves along the screw shaft 130 together with the nut 131; the elastic member 15 that is provided coaxially with the screw shaft 130 so as to be interposed between the nut 131 and the linear motion member 14; and the crank mechanism 17 that converts a linear motion of the linear motion member 14 into a rotational motion.

According to such a knee joint 1, since the elastic member 15 is provided coaxially with the screw shaft 130, it is possible to reduce the space in which parts are arranged and to make the knee joint 1 thinner as a whole, and thus weight reduction and miniaturization can be achieved. **In** addition, according to the knee joint 1, by achieving weight reduction and miniaturization, exterior parts with a design feature can be placed, and thus the design feature can be improved.

Also, according to the knee joint 1, since the elastic member 15 is interposed between the nut 131 and the linear motion member 14, the elastic member 15 absorbs manufacturing errors, mainly during the operation of the joint, and plays a greater role in absorbing external shocks. Also, when a lateral force is applied to the linear motion member 14 from the crank mechanism 17, the nut 131 must move up and down, but according to the knee joint 1, since the elastic member 15 is interposed between the nut 131 and the linear motion member 14, the elastic member 15 absorbs the lateral force, thereby enabling smooth movement of the linear motion member 14. As a result, unnecessary force to the nut 131 can be avoided, which in turn can lead to avoidance of locking of the screw shaft 130 and the nut 131 and reduction of the load on the nut 131.

Also, the knee joint 1 is provided with the speed reduction mechanism 12 that reduces the rotational speed of the motor 11 and transmits the power of the motor 11 to the ball screw 13, and the speed reduction mechanism 12 has the small pulley 120 which is fixed to the drive shaft 111 of the motor 11, the large pulley 121 which is fixed to the screw shaft 130 and has a larger diameter than the small pulley 120, and the annular belt 122 which is hooked to the small pulley 120 and the large pulley 121 and runs by rotation of the small pulley 120 to rotate the large pulley 121.

According to the knee joint 1, since the speed reduction mechanism 12 is provided, the torque of the motor 11 can be increased by the speed reduction mechanism 12 and transmitted to the ball screw 13. Therefore, since the necessary torque can be obtained with the low output motor 11, the motor 11 can be made smaller, and thus weight reduction and miniaturization can be achieved.

Also, according to the knee joint 1, since the knee joint 1 has the annular belt 122 that runs by the rotation of the small pulley 120 to rotate the large pulley 121, compared with the case in which gears engage and interlock, noise reduction can be achieved.

Also, the knee joint 1, as the elastic member 15, has the first elastic member 150 provided below the nut 131, and the second elastic member 151 provided above the nut 131, and the linear motion member 14 is a cylinder which has the nut 131, the first elastic member 150, and the second elastic member 151 built-in.

According to such a knee joint 1, with no relation to the operation of the motor 11, when the joint 172 connected to the crank mechanism 17 and the socket (omitted in the figures) connected to the socket connection portion 172a of the joint 172 operate, either the first elastic member 150 or the second elastic member 151 contract, so that the external shock or load transmitted through the crank mechanism 17 can be accurately absorbed, and the motor 11, the speed reduction mechanism 12, etc. can be protected. Also, according to the knee joint 1, the restoring force generated at the time of the contracted first elastic member 150 or the contracted second elastic member 151 being restored supports the rotational movement of the joint 172. Therefore, since the necessary power can be obtained with the low output motor 11, the motor 11 can be made smaller, and thus weight reduction and miniaturization can be achieved.

In addition, the knee joint 1 comprises the framework 10 supporting the screw shaft 130 and the cover (omitted in the figures) detachably attached to the framework 10.

According to such a knee joint 1, by attaching the cover (having an exterior structure that copies human curves) having a design feature that is appropriate for the user and situation, the design feature according to the user and situation can be ensured.

Also, in the knee joint 1, the framework 10 includes the lower support member 100 that rotatably supports the lower part of the screw shaft 130, the upper support member 101 that rotatably supports the upper part of the screw shaft 130, and the first pillar 102 that is provided in front of the screw shaft 130 so as to connect the lower support member 100 and the upper support member 101; and the knee joint 1 is provided with the rail 160 that is provided along the first pillar 102 and guides the movement of the linear motion member 14, and the guide block 161 that is fixed to the linear motion member 14 and slides along the rail 160.

According to such a knee joint 1, since the pair of the upper and lower support members 100, 101, the first pillar 102, etc. compose the framework 10, the framework 10 itself can be made smaller; therefore, weight reduction and miniaturization can be achieved. Also, according to the knee joint 1, since the knee joint 1 is provided with the rail 160 to guide the movement of the linear motion member 14, the linear motion member 14 can be moved smoothly.

Also, in the knee joint 1, the motor 11 is provided behind the screw shaft 130, and the framework 10 has the pair of left and right second pillars 103, 104 provided on the left and right sides of the screw shaft 130 or the motor 11 so as to connect the lower support member 100 and the upper support member 101.

According to such a knee joint 1, since the knee joint 1, as the framework 10, has the first pillar 102 and the pair of the left and right second pillars 103, 104, it can firmly support the weight of the user with the pair of the left and right second pillars 103, 104 together with the first pillar 102.

**In** addition, in the knee joint 1, the framework 10 has the base 105 that is provided below the lower support member 100 so as to face the lower support member 100 and is connected to the lower support member 100, and the covers 10a and 10b are attached to the left and right sides of the upper support member 101 and are attached to the lower side or the upper side of the base 105.

According to such a knee joint 1, since the covers 10a, 10b are attached to the left and right sides of the upper support member 101, when the user falls on his/her knees, the covers 10a, 10b can receive the force in the left-right direction that the covers 10a, 10b receive. Also, according to the knee joint 1, since the covers 10a, 10b are attached below or above the base 105, the covers 10a, 10b can receive the upward and downward forces that the covers 10a, 10b receive.

In addition, the artificial limb AL is provided with such a knee joint 1.

According to such an artificial limb AL**,** since the knee joint 1, which realizes weight reduction and miniaturization and also improves the design feature, is provided, the artificial limb AL having the knee joint 1 also can realize weight reduction and miniaturization, and can improve the design feature.

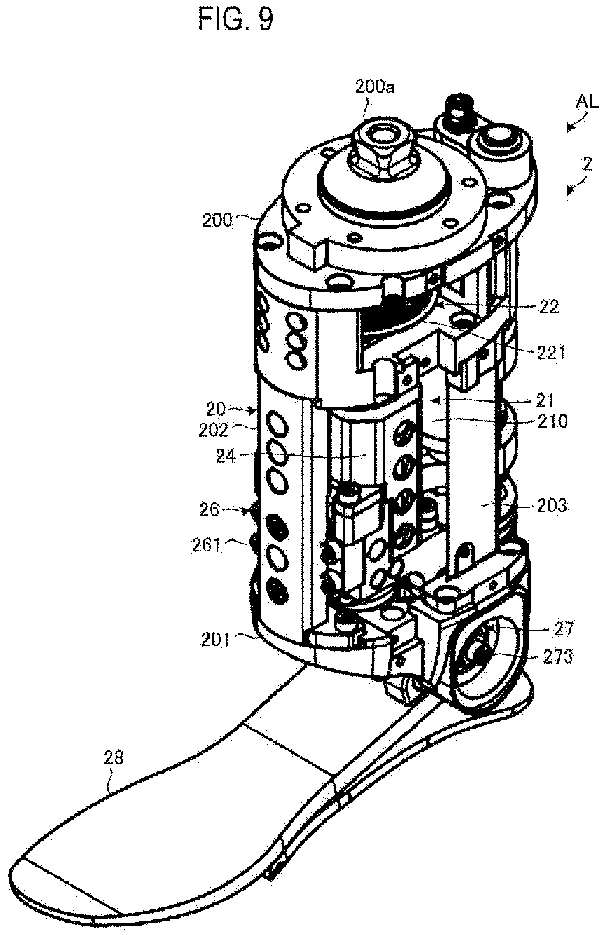

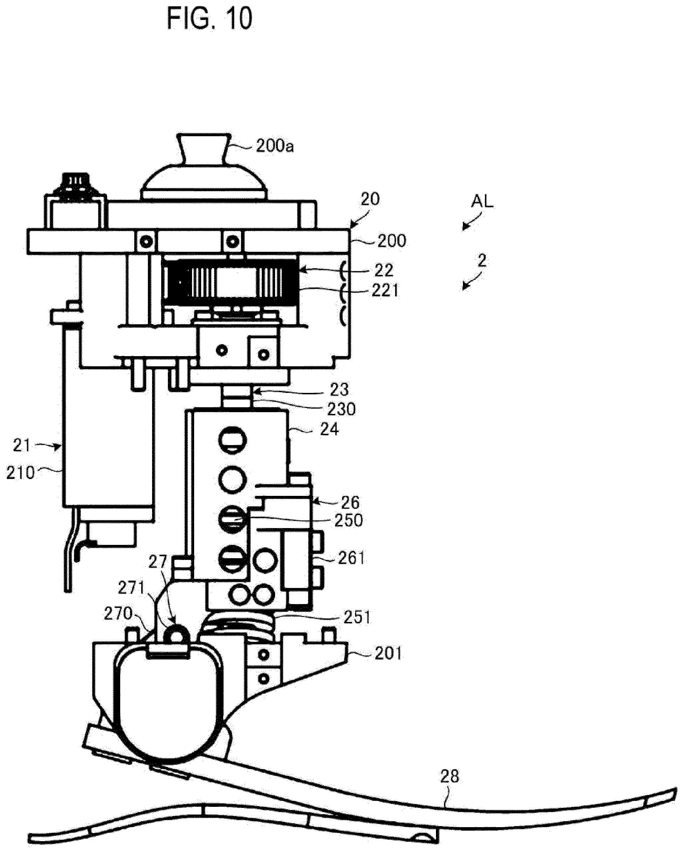

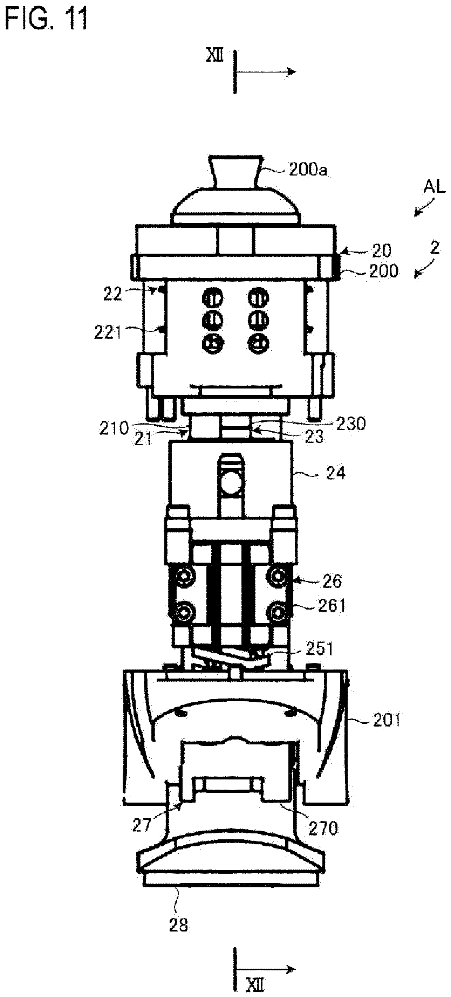

[Second Embodiment] Next, using FIGS.8 to 12, the configuration of the foot joint 2 of a second embodiment is described. FIG. 8 is a schematic perspective view illustrating the foot joint 2. FIG. 9 is a schematic perspective view illustrating the foot joint 2 with the condition of a cover 20a removed. FIG. 10 is a side view illustrating the foot joint 2 with the condition of pillars 202, 203 and the cover 20a removed. FIG. 11 is a front view illustrating the foot joint 2 with the condition of the pillars 202, 203 and the cover 20a removed. FIG. 12 is a cross-sectional view illustrating the foot joint 2 viewed in the arrow XII-XII direction shown in FIG. 11.

The foot joint (assist device) 2 shown in FIGS.8 to 12 constitutes the artificial limb AL together with a socket (omitted in the figures) fixed to a cut surface of a foot, a knee joint (e.g., the above-mentioned knee joint 1 (see FIGS.1 to 7)), a support portion 3 called a tube, etc., and assists the operation of a foot joint. The foot joint 2 is connected to a knee joint (e.g., the knee joint 1 (see FIGS.1 to 7)) via the support portion 3.

Specifically, the foot joint 2 comprises a framework 20, a motor 21, a speed reduction mechanism 22, a ball screw 23, a linear motion member 24, a first elastic member 250, a second elastic member 251, a guide member 26, a crank mechanism 27, a foot portion 28, etc.

The framework 20 forms the framework of the foot joint 2, and supports a screw shaft 230, etc. comprising the ball screw 23. This framework 20 has the cover 20a removably attached to the framework 20 itself. Specifically, the framework 20 comprises a pair of upper and lower support members 200, 201; a front first pillar 202; a left second pillar 203; a right second pillar paired with the left second pillar 203 (omitted in the figures), etc.

The pair of upper and lower support members 200, 201 are spaced apart from each other so as to face each other vertically, and are connected to each other by the front first pillar 202 and the pair of left and right second pillars 203 (the right second pillar is omitted in the figures). The upper support member 200 fixes a motor body 210 comprising the motor 21, and rotatably supports the upper part of the screw shaft 230 comprising the ball screw 23.

The upper support member 200 has a tube connection portion 200a on its upper surface. The tube connection 200a is used to connect the support portion 3, referred to as a tube, to the foot joint 2. The tube connection portion 200a, also referred to as a pyramid connector, can be connected to the support portion 3, referred to as a tube, using an existing method.

The lower support member 201 rotatably supports the lower part of the screw shaft 230 comprising the ball screw 23, and has the foot portion 28 relatively rotatably mounted by a rotation shaft 273.

The front first pillar 202, together with the pair of left and right second pillars 203 (the right second pillar is omitted in the figures), connects the pair of upper and lower support members 200, 201 to each other. The front first pillar 202 is spaced from each of the pair of left and right second pillars 203 (the right second pillar is omitted in the figures) and is provided in front of the screw shaft 230 along the screw shaft 230 comprising the ball screw 23. Also, the front first pillar 202 has a rail 260 comprising the guide member 26 fixed to its rear side.

The pair of left and right second pillars 203 (the right second pillar is omitted in the figures), together with the front first pillar 202, connect the pair of upper and lower support members 200, 201 to each other. The pair of left and right second pillars 203 (the right second pillar is omitted in the figures) is spaced from the front first pillar 202 and from each other, and is provided on the left and right sides of the screw shaft 23 or the motor 21 along the screw shaft 230 comprising the ball screw 23.

The cover 20a is removably attached to the framework 20 and covers the foot joint 2 from above and over the whole of the foot joint 2 except for the foot portion 28.

The motor 21 is the power source for the foot joint 2. The motor 21 is positioned on the rear and above the framework 20. Therefore, the motor 21 is provided behind the screw shaft 230. Specifically, the motor 21 comprises a motor body 210, a drive shaft 211, etc. The motor body 210, to make the drive shaft 211 be parallel to the screw shaft 230 comprising the ball screw 23, with the drive shaft 211 facing up, is fixed on the rear and below the upper support member 200 comprising the framework 20. The drive shaft 211 penetrates through the upper support member 200 comprising the framework 20, and is arranged to protrude above the upper support member 200.

The speed reduction mechanism 22 reduces the rotational speed of the motor 21 and transmits the power to the ball screw 23. The speed reduction mechanism 22 is positioned above the upper support member 200 comprising the framework 20. Specifically, the speed reduction mechanism 22 comprises a small pulley 220, a large pulley 221, an annular belt 222, etc.

The small pulley 220 has a smaller diameter than the large pulley 221. The small pulley 220 is fixed to the drive shaft 211 comprising the motor 21, and rotates by rotation of the motor 21. In addition, the small pulley 220 hooks the annular belt 222 together with the large pulley 221, and the belt 222 is run by rotation of the small pulley 220 itself.

The large pulley 221 has a larger diameter than the small pulley 220. The large pulley 221 is fixed to the upper end of the screw shaft 230 comprising the ball screw 23, and hooks the annular belt 222. Also, about the large pulley 221, the annular belt 222 runs to rotate the large pulley 221 itself, and the large pulley 221 itself rotates to rotate the screw shaft 230 comprising the ball screw 23.

The annular belt 222 is hooked to the small pulley 220 and the large pulley 221. About the annular belt 222, the small pulley 220 rotates to run the annular belt 222 itself, and the annular belt 222 itself runs to rotate the large pulley 221.

The ball screw 23 converts the rotational motion of the motor 21 transmitted by the speed reduction mechanism 22 into the linear motion. The ball screw 23 is provided inside the framework 20. Specifically, the ball screw 23 comprises the screw shaft 230, a nut 231, etc.

The screw shaft 230 is provided along each of the pillars 202, 203 (the second pillar on the right is omitted in the figures) so as to be parallel to each of the pillars 202, 203 (the second pillar on the right is omitted in the figures) comprising the framework 20, its upper part is rotatably supported by the upper support member 200, and its lower part is rotatably supported by the lower support member 201. The screw shaft 230 is fitted with a nut 231, and the screw shaft 230 itself rotates to move the nut 231 in the vertical direction. Also, the screw shaft 230 is coaxially provided with the linear motion member 24, the first elastic member 250, and the second elastic member 251.

The nut 231 is embedded in the screw shaft 230 and moves in the vertical direction as the screw shaft 230 rotates. The nut 231 is built into the linear motion member 24 together with the first elastic member 250, and the nut 231 itself moves upward to move the linear motion member 24 and the first elastic member 250 upward, and the nut 231 itself moves downward to move the linear motion member 24 downward via the first elastic member 250.

The linear motion member 24 is provided coaxially with the screw shaft 230 comprising the ball screw 23, and moves along the screw shaft 230 together with the nut 231 comprising the ball screw 23. The linear motion member 24 is a cylinder that the nut 231 comprising the ball screw 23 and the first elastic member 250 are built-in. Also, the linear motion member 24 is acted upon from the outside by the second elastic member 251 which is provided below it. In addition, the linear motion member 24 has a guide block 261 comprising the guide member 26 fixed to its front side, and moves along the rail 260 comprising the guide member 261. Furthermore, the linear motion member 24, on its lower part, has one end of a connecting rod 270 comprising the crank mechanism 27 relatively rotatably mounted with a first pin 271.

The first elastic member 250 is a coil spring provided coaxially with the screw shaft 230 comprising the ball screw 23 so as to be interposed between the nut 231 comprising the ball screw 23 and the linear motion member 24. The first elastic member 250 is provided below the nut 231 comprising the ball screw 23, and is built into the linear motion member 24 together with the nut 231.

The second elastic member 251 is a coil spring provided coaxially with the screw shaft 230 comprising the ball screw 23 so as to be interposed between the linear motion member 24 and the lower support member 201 comprising the framework 20, that is, so as to be provided above the lower support member 201 comprising the framework 20 and below the linear motion member 24. This second elastic member 251 acts on the linear motion member 24 from the outside.

The first elastic member 250 is pushed down by the nut 231 when the nut 231 comprising the ball screw 23 moves in the downward direction, and pushes down the linear motion member 24. When the linear motion member 24 moves down and pushes the second elastic member, the second elastic member 251 contracts itself. In addition, the first elastic member 250, without any relation to the fact that the nut 231 moves, when the linear motion member moves in the upward direction due to an external force, contracts by itself due to the external force transmitted through the linear motion member 24.

The second elastic member 251, when it is pushed by the linear member 24 as the linear member 24 moves in the downward direction along with the movement of the nut 231, contracts itself. In addition, the second elastic member 251, without any relation to the fact that the nut 231 moves, when linear motion member 24 moves in the downward direction due to an external force, contracts itself due to the external force transmitted through the linear motion member 24.

The guide member 26 is provided on the back of the front first pillar 202 comprising the framework 20, and guides the movement of the linear motion member 24. Specifically, the guide member 26 has the rail 260 and the guide block 26.

The rail 260 is fixed to the rear side of the front first pillar 202 comprising the framework 20 along the first pillar 202. The rail 260 is engaged with the guide block 261 so that the guide block 261 can slide, and guides the movement of the guide block 261. The guide block 261 is fixed in front of the linear motion member 24, and slides along the rail 260. Therefore, the rail 260, which guides the movement of the guide block 261, guides the movement of the linear motion member 24, which is fixed to the guide block 261.

The crank mechanism 27 is provided below the framework 20, converts the linear motion of the linear motion member 24 into the rotational motion, and transmits it to the foot portion 28 that serves as a ground portion. Specifically, the crank mechanism 27 comprises the connecting rod 270, a first pin 271, a second pin 272, a rotation shaft 273, etc.

The connecting rod 270 has one end relatively rotatably mounted to the lower part of the linear motion member 24 by the first pin 271, and the other end relatively rotatably mounted to the foot portion 28 by the second pin 272. The first pin 271 relatively rotatably attaches one end of the connecting rod 270 to the lower part of the linear motion member 24. The second pin 272 relatively rotatably attaches the other end of the connecting rod 270 to the foot portion 28. The rotation shaft 273 relatively rotatably attaches the foot portion 28 to the lower support member 201 comprising the framework 20.

The foot portion 28 is a grounding portion in the artificial limb AL. The foot portion 28 is relatively rotatably mounted to the lower support member 201 comprising the framework 20 by the rotation shaft 273, and has the connecting rod 270 relatively rotatably mounted by the second pin 272.

Next, using FIG. 10, the operation of the foot joint 2 is described.

First, the case in which the foot joint 2 drives the motor 21 is described. As the motor 21 is driven, the drive shaft 211 rotates. As the drive shaft 211 rotates, the small pulley 220 rotates in unison with the drive shaft 211. As the small pulley 220 rotates, the annular belt 222 runs. As the annular belt 222 runs, the large pulley 221 rotates. As the large pulley 221 rotates, the screw shaft 230 rotates in unison with the large pulley 221.

As the screw shaft 230 rotates, according to the direction of rotation of the screw shaft 230, the nut 231 moves up or down. When the nut 231 moves in the upward direction, in unison with the nut 231, together with the first elastic member 250, the linear motion member 24 moves in the upward direction along the rail 260. When the nut 231 moves in the downward direction, in unison with the nut 231, together with the first elastic member 250, the linear motion member 24 moves in the downward direction along the rail 260.

When the linear motion member 24 moves in the upward direction, the connecting rod 270 moves in the upward direction. When the linear motion member 24 moves in the downward direction, the connecting rod 270 moves in the downward direction. When the connecting rod 270 moves in the upward direction, the foot portion 28 rotates centering the rotation shaft 273 in the direction of the foot joint to plantarflex. When the connecting rod 270 moves in the downward direction, the foot portion 28 rotates centering the rotation shaft 273 in the direction of the foot joint to dorsiflex.

Next, the case in which an external shock or load is applied to the foot joint 2 is described. When an external shock or load is applied in the direction of the foot joint to plantarflex, in the direction of the foot joint to plantarflex, the foot portion 28 rotates centering the rotation shaft 273. When an external shock or load is applied in the direction of the foot joint to dorsiflex, in the direction of the foot joint to dorsiflex, the foot portion 28 rotates centering the rotation shaft 273. When the foot portion 28 rotates centering the rotation shaft 273 in the direction of the foot joint to plantarflex, the connecting rod 270 moves in the upward direction. When the foot portion 28 rotates centering the rotation shaft 273 in the direction of the foot joint to dorsiflex, the connecting rod 270 moves in the downward direction. When the connecting rod 270 moves in the upward direction, the linear motion member 24 moves in the upward direction. When the connecting rod 270 moves in the downward direction, the linear motion member 24 moves in the downward direction.

When the linear motion member 24 moves in the upward direction, the first elastic member 250 contracts. When the linear motion member 24 moves in the downward direction, the second elastic member 251 contracts. When the contracted first elastic member 250 is restored, the restoring force by the first elastic member 250 generated at that time supports the movement of the foot portion 28. When the contracted second elastic member 251 is restored, the restoring force by the second elastic member 251 generated at that time supports the movement of the foot portion 28.

Continuing further, the foot joint 2 in the case of transitioning from forward movement of the trunk to the kicking out motion is described. When the trunk moves forward, an external load is applied in the direction of the foot joint to dorsiflex, and in the direction of the foot joint to dorsiflex, the foot portion 28 rotates centering the rotation shaft 273. As the foot portion 28 rotates centering the rotation shaft 273 in the direction of the foot joint to dorsiflex, the connecting rod 270 moves downward. As the connecting rod 270 moves in the downward direction, the linear motion member 24 moves in the downward direction. As the linear motion member 24 moves in the downward direction, the second elastic member 251 contracts.

Thereafter, a transition is made to the kicking out motion. When the kicking out motion is performed, the motor 21 is driven so that the foot joint plantarflexes. By driving the motor 21 so that the foot joint plantarflexes, the linear motion member 24 moves up along the rail 260. At this time, the second elastic member 251, which had been contracted, is restored, and the restorative force generated by the second elastic member 251 at that time pushes up the linear motion member 24, thereby supporting the kicking out motion of the foot portion 28. Thereby, the output of the motor 21 can be reduced. Since the necessary power can be obtained with the low output motor 21, the motor 21 and the battery can be made smaller, and thus the weight reduction and miniaturization can be achieved.

Thus, the foot joint 2 is an assist device that assists the operation of a foot joint, and comprises the ball screw 23 that converts the rotational motion of the screw shaft 230 rotated by the motor 21 into the linear motion of the nut 231; the framework 20 having the lower support member 201 that rotatably supports the lower part of the screw shaft 230; the linear motion member 24, which is provided coaxially with the screw shaft 230 and moves along the screw shaft 230 together with the nut 231; the first elastic member 250 provided coaxially with the screw shaft 230 so as to be interposed between the nut 231 and the linear member 24; and the second elastic member 251 provided coaxially with the screw shaft 230 so as to be interposed between the linear member 24 and the lower support member 201; and the crank mechanism 27 that converts the linear motion of the linear motion member 24 into the rotational motion.

According to such a foot joint 2, since the first elastic member 250 and the second elastic member 251 are provided coaxially with the screw shaft 230, the space where the parts are arranged can be made smaller and the entire foot joint 2 can be made thinner, and thus, weight reduction and miniaturization can be achieved. Further, according to the foot joint 2, by achieving weight reduction and miniaturization, exterior parts having a design feature (e.g., the cover 20a) can be arranged, and thus, the design feature can be improved.

Also, according to the foot joint 2, since the first elastic member 250 is interposed between the nut 231 and the linear motion member 24, and the second elastic member 251 is interposed between the linear motion member 24 and the lower support member 201, the first elastic member 250 and the second elastic member 251 absorb manufacturing errors, and mainly during the joint movement, the role of absorbing external shocks becomes larger. **In** addition, when a lateral force is applied to the linear motion member 24 from the crank mechanism 27, the nut 231 must move up and down, but according to the foot joint 2, the first elastic member 250 is interposed between the nut 231 and the linear motion member 24, and the second elastic member 251 is interposed between the linear motion member 24 and the lower support member 201; therefore, the first elastic member 250 and the second elastic member 251 absorb the lateral force so that smooth movement of the linear motion member 24 can be achieved. As a result, the action of an unnecessary force on the nut 231 can be avoided, which in turn can lead to the avoidance of locking of the screw shaft 230 and the nut 231 and the reduction of the load on the nut 231.

**In** addition, the foot joint 2 is provided with a speed reduction mechanism 22 that reduces the rotational speed of the motor 21 and transmits the power of the motor 21 to the ball screw 23, and the speed reduction mechanism 22 has the small pulley 220 fixed to the drive shaft 211 of the motor 21; the large pulley 221 fixed to the screw shaft 230 and having a larger diameter than the small pulley 220, and the annular belt 222 which is hooked to the small pulley 220 and the large pulley 221 and runs by rotation of the small pulley 220 to rotate the large pulley 221.

According to the foot joint 2, since the foot joint 2 is provided with the speed reduction mechanism 22, the torque of the motor 21 can be increased by the speed reduction mechanism 22 and transmitted to the ball screw 23. Therefore, since the necessary torque can be obtained with the low output motor 21, the motor 21 can be made smaller, and thus weight reduction and miniaturization can be achieved.

Also, according to the foot joint 2, since the foot joint 2 has the annular belt 222 which is run by the rotation of the small pulley 220 to rotate the large pulley 221, compared with the case in which gears engage and interlock, noise reduction can be achieved.

Also, in the foot joint 2, the linear motion member 24 is a cylinder that incorporates the nut 231 and the first elastic member 250; the first elastic member 250 is provided below the nut 231, and the second elastic member 251 is provided below the linear motion member 24.

According to such a foot joint 2, when the foot is dorsiflexed, the linear motion member 24 moves downward by the crank mechanism 27, and the second elastic member 251 contracts, so that the external shock and load transmitted through the crank mechanism 27 can be accurately absorbed, and the motor 21 and the speed reduction mechanism 22, etc. can be protected. **In** addition, when the foot is kicked out, the restoring force generated at the time of the contracted second elastic member 251 being restored supports the kicking out movement of the foot portion 28. As a result, the output of the motor 21 can be suppressed, and the necessary power can be obtained with the low output motor 21, so that the motor 21 can be made smaller, and thus weight reduction and miniaturization can be achieved.

Also, according to the foot joint 2, without any relation to the operation of the motor 21, when the foot portion connected to the crank mechanism 27 operates, the first elastic member 250 contracts, thereby accurately absorbing external shocks and loads transmitted through the crank mechanism 27, and protecting the motor 21 and the speed reduction mechanism 22, etc. Also, according to the foot joint 2, the restoring force generated at the time of the contracted first elastic member 250 being restored supports the operation of the foot portion 28. Therefore, since the necessary power can be obtained with the low output motor 21, the motor 21 can be made smaller, and thus weight reduction and miniaturization can be achieved.

Also, the foot joint 2 is provided with the cover 20a that is removably attached to the framework 20.

According to such a foot joint 2, as the cover 20a (a cover having an exterior structure that copies human curves) with a design feature that is appropriate for the user or situation is attached, the design feature according to the user and situation can be ensured.

Also, in the foot joint 2, the framework 20 has the upper support member 200 that rotatably supports the upper part of the screw shaft 230 and the first pillar 202 that is provided in front of the screw shaft 230 so as to connect the lower support member 201 and the upper support member 200; and the foot joint 2 is provided by the first pillar 202 and comprises the rail 260 that guides the movement of the linear motion member 24, and the guide block 261 that is fixed to the linear motion member 24 and slides along the rail 260.

According to such a foot joint 2, since the pair of upper and lower support members 200, 201, the first pillar 202, etc. compose the framework 20, the framework 20 itself can be made smaller, and thus weight reduction and miniaturization can be achieved. Also, according to the foot joint 2, since the rail 260 to guide the movement of the linear motion member 24 is provided, the linear motion member 24 can be moved smoothly.

Also, in the foot joint 2, the motor 21 is provided behind the screw shaft 230, and the framework 20 has the pair of left and right second pillars 203 (the right second pillar is omitted in the figures) provided on the left and right sides of the screw shaft 230 or the motor 21 so as to connect the lower support member 201 and the upper support member 200.

According to such a foot joint 2, since the foot joint 2 has the first pillar 202 and the pair of left and right second pillars 203 (the right second pillar is omitted in the figures) as the framework 20, the weight of the user can be firmly supported by the first pillar 202 as well as the pair of left and right second pillars 203 (the right second pillar is omitted in the figures).

Also, in the foot joint 2, the cover 20a is provided in such a way that it is covered from above the upper support member 200 and wrapped around to below the lower support member 201, and is elastic and waterproof.

According to such a foot joint 2, the inside can be protected by the cover 20a, which has elastic and waterproof properties.

In addition, the artificial limb AL is provided with such a foot joint 2.

According to such an artificial limb AL, since the foot joint 2, which realizes weight reduction and miniaturization as well as improved the design feature, is provided, the artificial limb AL having the foot joint 2 can also achieve weight reduction and miniaturization, and thus can improve the design feature.

The present invention is not limited to the above-described embodiments, and various variations are possible within the scope of not deviating from the purpose and technical idea thereof. That is, the position, size, length, quantity, shape, material, etc. of each configuration can be changed as appropriate.

For example, in the foot joint 2 of a second embodiment described above, the case in which the first elastic member 250 is provided only below the nut 231 is described as an example, but the present invention is not limited thereto; in the foot joint 2, the first elastic member 250 may be provided at least either above the nut 231 or below the nut 231. That is, in the foot joint 2, the first elastic member 250 may be provided only above the nut 231, or may be provided both above and below the nut 231.

Alternatively, in the foot joint 2 of a second embodiment described above, the case in which the first elastic member 250 is provided is described as an example, but the present invention is not limited thereto, and the foot joint 2 may not comprise the first elastic member 250.

In the case where the first elastic member 250 is not provided, the foot joint 2 is an assist device that assists the operation of a foot joint, and comprises the ball screw 23 that converts a rotational motion of the screw shaft 230 rotated by the motor 21 into a linear motion of the nut 231; the framework 20 that has the lower support member 201 rotatably supporting the lower part of the screw shaft 230; the linear motion member 24 that is coaxially provided with the screw shaft 230 and moves along the screw shaft 230 together with the nut 231; the second elastic member (elastic member) 251 that is coaxially provided with the screw shaft 230 so as to be interposed between the linear motion member 24 and the lower support member 201; and the crank mechanism 27 that converts the linear motion of the linear motion member 24 into the rotational motion.

According to such a foot joint 2, since the second elastic member (elastic member) 251 is provided coaxially with the screw shaft 230, the space where the parts are arranged can be made smaller, and consequently, weight reduction and miniaturization can be achieved. In addition, according to such a foot joint 2, by achieving weight reduction and miniaturization, exterior parts having a design feature can be arranged, and thus, the design feature can be improved.

Also, according to such a foot joint 2, when the foot is dorsiflexed, the linear motion member 24 moves down by the crank mechanism 27, and the second elastic member (elastic member) 251 contracts, so that the external shock and load transmitted via the crank mechanism 27 can be accurately absorbed, and the motor 21, the speed reduction mechanism 22, etc. can be protected. **In** addition, when the foot is kicked out, the restoring force generated at the time of the contracted second elastic member (elastic member) 251 being restored supports the kicking out operation of the foot portion 28. For this reason, the output of the motor 21 can be suppressed, and the necessary power can be obtained with the low output motor 21, so that the motor 21 can be made smaller, and thus weight reduction and miniaturization can be achieved.

Also, in the knee joint 1 of a first embodiment described above, the case in which a coil spring is provided as the elastic member 15 coaxially provided with the screw shaft 130 is described as an example, but the present invention is not limited thereto; and the knee joint 1, as the elastic member 15 coaxially provided with the screw shaft 130, instead of a coil spring, may comprise a rubber-based or urethane-based cushion, or a disc spring.

Also, in the foot joint 2 of a second embodiment described above, the case in which a coil spring is provided as the first elastic member 250 coaxially provided with the screw shaft 230 is described as an example, but the present invention is not limited thereto; and the foot joint 2, as the first elastic member 250 coaxially provided with the screw shaft 230, instead of a coil spring, may comprise a rubber-based or urethane-based cushion, or a disc spring.

Also, in the foot joint 2 of a second embodiment described above, the case in which a coil spring is provided as the second elastic member 251 provided coaxially with the screw shaft 230 is described as an example, but the present invention is not limited thereto; and the foot joint 2, as the second elastic member 251 provided coaxially with the screw shaft 230, instead of a coil spring, may comprise a rubber-based or urethane-based cushion, or a disc spring.

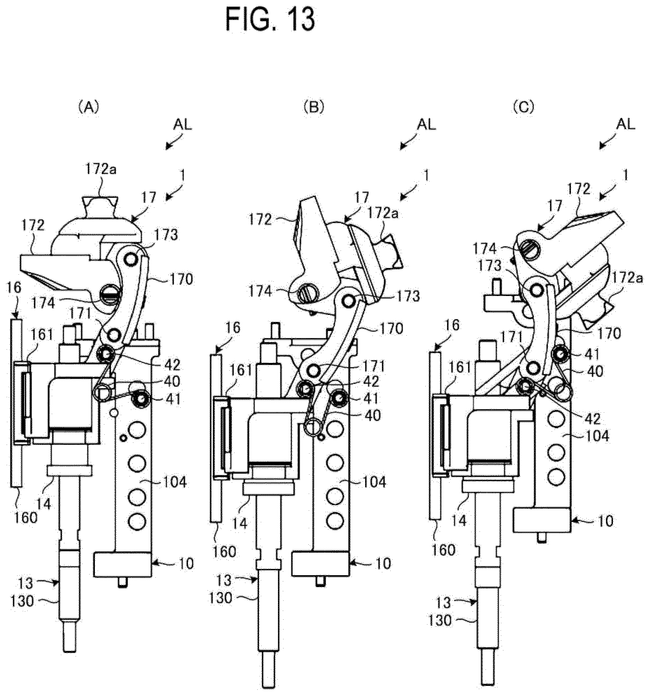

Also, as shown in FIG. 13, the knee joint 1 of a first embodiment described above may be provided with a third elastic member 40 that assists in flexion and extension of the knee joint. FIG. 13, in a variation in which the third elastic member 40 is added to the knee joint 1, is a side view illustrating the condition of the pillars 102, 103, the covers 10a, 10b, and the battery block 18 removed. FIG. 13(A) shows the condition in which the knee joint is extended. FIG. 13(B) shows the condition in which the knee joint is bent halfway (about 70°). FIG. 13(C) shows the condition in which the knee joint is fully bent.

The knee joint 1 shown in FIGS.13(A) to 13(C) is a variation in which the third elastic member 40 is added to the knee joint 1 according to a first embodiment. The knee joint 1 according to this variation, in addition to the configuration of the knee joint 1 according to a first embodiment, comprises the third elastic member 40, a third pin 41 (first support portion), and a fourth pin (second support portion) 42.

The third elastic member 40 is a toggle spring interposed between the third pin 41 and the fourth pin 42. Specifically, the third elastic member 40 is rotatably supported at one end by the third pin 41 and rotatably supported at the other end by the fourth pin 42. This third elastic member 40 is compressed as the third pin 41 and the fourth pin 42 approach each other, and generates the restoring force in the direction in which the third pin 41 and the fourth pin 42 move away from each other.

The third pin 41 is built between the pair of left and right second pillars 103, 104, and rotatably supports one end of the third elastic member 40. The fourth pin 42 is provided above the linear motion member 14 and near directly below the first pin 171 so as to be parallel to the third pin 41, and rotatably supports the other end of the third elastic member 40.

The third pin 41 and the fourth pin 42, when their relative heights coincide, i.e., when they are lined up in front of and behind each other in the horizontal direction, become closest to each other. In other words, as shown in FIG. 13(B), when the knee joint is bent halfway, the relative heights are close, and the pins are close to each other. In addition, the third pin 41 and the fourth pin 42 are separated from each other as their relative heights are different. That is, when the knee joint is extended as shown in FIG. 13(A), the relative heights are different, and the pins become distant from each other. Also, when the knee joint is fully bent as shown in FIG. 13(B), the relative heights become different, and they are distanced from each other.

Thus, the third pin 41 and the fourth pin 42, in the process of transitioning from the condition in which the knee joint is extended to the condition in which the knee joint is fully bent, gradually approach each other, and then move away from each other (FIG. 13(A) → FIG. 13(B) → FIG. 13(C)). In addition, the third pin 41 and the fourth pin 42, in the process of transitioning from the condition in which the knee joint is fully bent to the condition in which the knee joint is extended, gradually approach each other, and then gradually move away from each other.

For this reason, the third elastic member 40 interposed between the third pin 41 and the fourth pin 42, in the process of transitioning from the condition in which the knee joint is extended to the condition in which the knee joint is fully bent (FIG. 13(A) → FIG. 13(B) → FIG. 13(C)), is compressed and generates the restoring force in the direction in which the third pin 41 and the fourth pin 42 move away from each other. In other words, the third elastic member 40 is compressed until the condition in which the knee joint is bent by a little more than the approximately 70° bent shown in FIG. 13(B), thereby generating the restoring force in the direction that prevents the knee joint from being bent, and then when the knee joint is further bent from the condition in which the knee joint is bent by a little more than the approximately 70° bent shown in FIG. 13(B), by releasing the energy stored by being compressed up to now, the restoring force is generated in the direction that assists in flexing the knee joint, and by giving the downward force to the linear motion member 14, the linear motion member 14 becomes easy to move down. This causes the knee joint to bend through the crank mechanism 17.

In addition, the third elastic member 40 interposed between the third pin 41 and the fourth pin 42, in the process of transitioning from the condition in which the knee joint is fully bent to the condition in which the knee joint is extended (FIG. 13(C) → FIG. 13(B) → FIG. 13(A)), is compressed and generates the restoring force in the direction in which the third pin 41 and the fourth pin 42 move away from each other. In other words, the third elastic member 40 is compressed until the condition slightly before reaching the condition of approximately 70° flexion shown in FIG. 13(B), thereby generating the restoring force in the direction that prevents the knee joint from being extended, and then when the knee joint is further compressed from the condition slightly before reaching the condition of approximately 70° flexion shown in FIG. 13(B), by releasing the energy stored by being compressed up to now, the restoring force is generated in the direction that assists in extending the knee joint, and by providing the upward force to the linear motion member 14, the linear motion member 14 becomes easy to move up. This causes the knee joint to be extended through the crank mechanism 17.

Thus, the knee joint 1 according to the variation comprises the third pin 41 provided in the framework 10; the fourth pin 42 that moves between the position above the third pin 41 and the position below the third pin 41 by moving together with the linear motion member 14; and the third elastic member 40 whose one end is rotatably supported by the third pin 41, and the other end is rotatably supported by the fourth pin 42.

According to the knee joint 1 according to the variation, when the knee is extended, the third elastic member 40 assists the movement of the knee joint, so that the necessary power can be obtained with the low output motor 11; therefore, the motor 11 can be made smaller, and thus weight reduction and miniaturization can be achieved. Also, according to the knee joint 1 according to the variation, when the battery runs out, extension can be assisted by the third elastic member 40 in accordance with the inertia, so that the knee can be extended more easily. Thereby, in addition to being able to cope with knee extension when walking quickly, knee buckling caused by not being able to extend sufficiently can be prevented.

Also, in the variation of the knee joint 1, the case in which a toggle spring is provided as the third elastic member 40 is described as an example, but the present invention is not limited thereto, and a coil spring may be provided instead of a toggle spring as the third elastic member 40.

Also, as shown in FIG. 14, the knee joint 1 of a first embodiment described above may be provided with a speed reduction mechanism 50 instead of the speed reduction mechanism 12. FIG. 14 is a diagram illustrating a variation in which the speed reduction mechanism 12 (see FIG. 5, etc.) in the knee joint 1 is changed to the speed reduction mechanism 50. FIG. 14(A) is a cross-sectional view in the direction of the arrow XIVA-XIVA shown in FIG. 14(B). FIG. 14(B) is a bottom view with the condition of parts lower than the speed reduction mechanism 50 removed.

The knee joint 1 shown in FIGS.14(A) and 14(B) is a variation in which the speed reduction mechanism 12 in the knee joint 1 according to a first embodiment is changed to the speed reduction mechanism 50. The knee joint 1 according to this variation has the speed reduction mechanism 50 instead of the speed reduction mechanism 12 in the knee joint 1 according to a first embodiment.

The speed reduction mechanism 50 reduces the rotational speed of the motor 11 and transmits it to the ball screw 13. The speed reduction mechanism 50 is positioned below the lower support member 100 comprising the framework 10. Specifically, the speed reduction mechanism 50 comprises a small gear 51, a rotation shaft 52, a two-stage gear 53, a large gear 54, etc.

The small gear 51 has a smaller diameter than the larger diameter gear (sign is omitted) in the two-stage gear 53, and also has a smaller diameter than the large gear 54. The small gear 51 is fixed to the drive shaft 111 comprising the motor 11, and rotates by rotation of the motor 11. Also, the small gear 51 is engaged with a larger diameter gear (sign is omitted) in the two-stage gear 53, and the small gear 51 itself rotates to rotate the two-stage gear 53.

The rotation shaft 52 is fixed to the lower support member 100 comprising the framework 10 so as to be parallel to each of the drive shaft 111 comprising the motor 11 and the screw shaft 130 comprising the ball screw 13. The rotation shaft 52 rotatably supports the two-stage gear 53.

The two-stage gear 53 rotates in unison with a gear of relatively larger diameter (sign is omitted) and a gear of relatively smaller diameter (sign is omitted). The relatively larger diameter gear (sign is omitted) in the two-stage gear 53 has a larger diameter compared to the smaller gear 51. The relatively small gear (sign is omitted) in the two-stage gear 53 has a smaller diameter than the large gear 54. The two-stage gear 53 is rotatably supported by the rotation shaft 52. Also, in the two-stage gear 53, the relatively larger diameter gear (sign is omitted) is engaged with the small gear 51, and the relatively smaller diameter gear (sign is omitted) is engaged with the large gear 54, so that the two-stage gear 53 itself rotates by rotation of the small gear 51, and the two-stage gear 53 itself rotates to rotate the large gear 54.

The large gear 54 has a larger diameter compared to the smaller diameter gear (sign is omitted) in the two-stage gear 53. The large gear 54 is fixed to the screw shaft 130 comprising the ball screw 13, and is engaged with the smaller diameter gear (sign is omitted) in the two-stage gear 53. Also, the large gear 54 itself rotates as the two-stage gear 53 rotates, and the rotation of the large gear 54 itself rotates the screw shaft 130 comprising the ball screw 13.

Thus, the knee joint 1 according to the variation comprises the speed reduction mechanism 50 that reduces the rotation speed of the motor 11 and transmits the power of the motor 11 to the ball screw 13, and the speed reduction mechanism 50 has the small gear 51 fixed to the drive shaft 111 of the motor 11, and the large gear 54 that is fixed to the screw shaft 130, that has a larger diameter compared to the small gear 51, and that interlocks with the small gear 51 via the two-stage gear (other gear) 53.

According to the knee joint 1 according to the variation, since the speed reduction mechanism 50 is provided, the torque of the motor 11 can be increased by the speed reduction mechanism 50 and transmitted to the ball screw 13. Therefore, since the necessary torque can be obtained with the low output motor 11, the motor 11 can be made smaller, and thus weight reduction and miniaturization can be achieved.

Also, according to the knee joint 1 according to the variation, since the small gear 51 and the large gear 54 are indirectly engaged and interlocked, the power can be transmitted reliably, and energy loss is minimal. Therefore, since the necessary torque can be obtained with the low output motor 11, the motor 11 can be made smaller, and thus weight reduction and miniaturization can be achieved. **In** addition, as in the knee joint 1 according to the variation, when gears are employed, since the friction between the gears is small, high back-drivability can be obtained. Thus, the knee joint 1 according to the variation, by obtaining high back-drivability, when the battery runs out, as a passive artificial limb, by extending and flexing the knee by inertia, can continuously walk even when the battery runs out.

Also, in the knee joint 1 according to the variation, the case in which the small gear 51 and the large gear 54 are indirectly engaged and interlocked is described as an example, but the present invention is not limited thereto, and the small gear 51 and the large gear 54 may be directly engaged and interlocked.

Also, in the above-mentioned first embodiment, the case of the knee joint 1 is described as an example, but the present invention is not limited thereto, and as long as the joint adopts the similar configuration, the knee joint may be a foot joint. Furthermore, in the above-mentioned first embodiment, the case of a knee joint 1 comprising the artificial limb AL is described as an example, but the present invention is not limited thereto, and as long as the joint adopts the similar configuration, the knee joint may be a joint for other purposes, such as a power suit joint, etc.

Also, in the above-mentioned second embodiment, the case of the foot joint 2 is described as an example, but the present invention is not limited thereto, and as long as the joint adopts the similar configuration, the foot joint may be a knee joint. Furthermore, in the above-mentioned second embodiment, the case of the foot joint 2 comprising the artificial limb AL is described as an example, but the present invention is not limited thereto, and as long as the joint adopts the similar configuration, the foot joint may be a joint for other purposes, such as a power suit joint, etc.

### DESCRIPTION OF SIGNS

AL Artificial limb
1 Knee joint (assist device)
10 Framework
100 Lower support member
101 Upper support member
102 First pillar
103, 104 Second pillars
105 Base
105a Tube connection portion
106, 107, 108, 109 Connecting member
10a Upper cover (cover)
10b Lower cover (cover)
11 Motor
110 Motor body
111 Drive shaft
12 Speed reduction mechanism (not part of the invention)
120 Small pulley
121 Large pulley
122 Annular belt
13 Ball screw
130 Screw shaft
131 Nut
14 Linear motion member (cylinder)
15 Elastic member
150 First elastic member
151 Second elastic member
16 Guide member
160 Rail
161 Guide block
17 Crank mechanism
170 Connecting rod
171 First pin
172 Joint
172a Socket connection portion
173 Second pin
174 Rotation shaft
18 Battery box
2 Foot joint (assist device)
20 Framework
200 Upper support member
200a Tube connection portion
201 Lower support member
202 First pillar
203 Second pillar
20a Cover
21 Motor
210 Motor body
211 Drive shaft
22 Speed reduction mechanism
220 Small pulley
221 Large pulley
222 Annular belt
23 Ball screw
230 Screw shaft
231 Nut
24 Linear motion member
250 First elastic member
251 Second elastic member (elastic member)
26 Guide member
260 Rail
261 Guide block
27 Crank mechanism
270 Connecting rod
271 First pin
272 Second pin
273 Rotation shaft
28 Foot portion
3 Support portion (tube)
40 third elastic member
41 third pin (first support portion)
42 fourth pin (second support portion)
50 Speed reduction mechanism
51 Small gear
52 Rotation shaft
53 Two-stage gear
54 Large gear

## Claims

1. An assist device (1; 2) to assist a movement of a joint (172) comprising:
a ball screw (13; 23) that is configured to convert a rotational motion of a screw shaft (130; 230) rotated by a motor (11; 21) into a linear motion of a nut (131; 231);
a linear motion member (14; 24) that is provided coaxially with the screw shaft (130; 230) and configured to move along the screw shaft (130; 230) with the nut (131; 231);
an elastic member (150, 151; 250, 251) that is provided coaxially with the screw shaft (130; 230) so as to interpose between the nut (131; 231) and the linear motion member (14; 24); and
a speed reduction mechanism (50) that is configured to reduce a rotational speed of the motor (11; 21) and to transmit a power of the motor (11; 21) to the ball screw (13; 23),
**characterized by**
a crank mechanism (17; 27) that is configured to convert the linear motion of the linear motion member (14; 24) into a rotational motion,wherein
the speed reduction mechanism (50) comprises:
a small gear (51) fixed to a drive shaft of the motor (11; 21); and
a large gear (54) fixed to the screw shaft (130; 230), having a larger diameter than the small gear (51), and interlocking with the small gear (51) via another gear (53) or directly engaging with the small gear (51).

2. The assist device (1; 2) according to claim 1 or 2, wherein
the elastic member(150, 151; 250, 251) comprises:
a first elastic member (150; 250) provided below the nut (131; 231); and
a second elastic member (151; 251) provided above the nut (131; 231), and
the linear motion member (14; 24) is a cylinder that incorporates the nut (131; 231), the first elastic member (150; 250), and the second elastic member (151; 251).

3. The assist device (1; 2) according to any of claims 1 to 3, comprising:
a framework (10; 20) that supports the screw shaft (130; 230); and
a cover (10a, 10b; 20a) that is detachably attached to the framework (10; 20).

4. The assist device (1; 2) according to claim 4, wherein
the framework (10; 20) comprises:
a lower support member (100; 201) that rotatably supports a lower part of the screw shaft (130; 230);
an upper support member (101; 200) that rotatably supports an upper part of the screw shaft (130; 230); and
a first pillar (102; 202) that is provided in front of the screw shaft (130; 230) to connect the lower support member (100; 201) to the upper support member (101; 200), and
the assist device (1) comprises:
a rail (160; 260) that is provided along the first pillar (102; 202) and configure to guide the movement of the linear motion member (14; 24); and
a guide block (161; 261) that is fixed to the linear motion member (14; 24) and configured to slide along the rail (160; 260).

5. The assist device (1; 2) according to claim 5, wherein
the motor (11; 21) is provided behind the screw shaft (130; 230), and
the framework (10; 20) comprises a pair of left and right second pillars (103, 104; 203) provided to left and right sides of the screw shaft (130; 230) or the motor (11; 21) so as to connect the lower support member (100; 201) to the upper support member (101; 200).

6. The assist device (1) according to claims 5 or 6, wherein
the framework (10) comprises a base (105) that is provided below the lower support member (100) to face the lower support member (100) and is connected to the lower support member (100), and
the cover (10a, 10b) is attached to left and right sides of the upper support member (101) and attached below or above the base.

7. The assist device (1) according to any of claims 4 to 7, comprising:
a first support portion (41) that is provided on the framework (10; 20);
a second support portion (42) that is configured to move in unison with the linear motion member (14; 24) to move between a position above the first support portion (41) and a position below the first support portion (41); and
a third elastic member (40) in which one end is rotatably supported by the first support portion (41) and the other end is rotatably supported by the second support portion (42).

8. An artificial limb comprising the assist device (1; 2) according to any of claims 1 to 8.

## Patentansprüche

1. Unterstützungsvorrichtung (1; 2) zur Unterstützung einer Bewegung eines Gelenks (172) mit:
einer Kugelumlaufspindel (13; 23), die so konfiguriert ist, dass sie eine Drehbewegung einer Spindelwelle (130; 230), die von einem Motor (11; 21) gedreht wird, in eine lineare Bewegung einer Mutter (131; 231) umwandelt;
einem Linearbewegungselement (14; 24), das koaxial mit der Schraubenwelle (130; 230) bereitgestellt ist und so konfiguriert ist, dass es sich zusammen mit der Mutter (131; 231) entlang der Schraubenwelle (130; 230) bewegt;
einem elastischen Element (150, 151; 250, 251), das koaxial mit der Schraubenwelle (130; 230) bereitgestellt ist, um zwischen der Mutter (131; 231) und dem Linearbewegungselement (14; 24) angeordnet zu werden; und
einem Untersetzungsmechanismus (50), der konfiguriert ist, eine Drehzahl des Motors (11; 21) zu reduzieren und eine Leistung des Motors (11; 21) auf die Kugelumlaufspindel (13; 23) zu übertragen, **gekennzeichnet durch**
einen Kurbelmechanismus (17; 27), der konfiguriert ist, die lineare Bewegung des Linearbewegungselements (14; 24) in eine Drehbewegung umzuwandeln, wobei
der Untersetzungsmechanismus (50) hat:
ein kleines Zahnrad (51), das an einer Antriebswelle des Motors (11; 21) befestigt ist; und
ein großes Zahnrad (54), das an der Schraubenwelle (130; 230) befestigt ist, einen größeren Durchmesser als das kleine Zahnrad (51) aufweist und mit dem kleinen Zahnrad (51) über ein anderes Zahnrad (53) ineinandergreift oder direkt mit dem kleinen Zahnrad (51) in Eingriff steht.

2. Unterstützungsvorrichtung (1; 2) gemäß Anspruch 1 oder 2, wobei das elastische Element (150, 151; 250, 251) hat:
ein erstes elastisches Element (150; 250), das unterhalb der Mutter (131; 231) bereitgestellt ist; und
ein zweites elastisches Element (151; 251), das oberhalb der Mutter (131; 231) bereitgestellt ist, und
das Linearbewegungselement (14; 24) ein Zylinder ist, der die Mutter (131; 231), das erste elastische Element (150; 250) und das zweite elastische Element (151; 251) umfasst.

3. Unterstützungsvorrichtung (1; 2) gemäß einem der Ansprüche 1 bis 3, mit:
einer Rahmenkonstruktion (10; 20), die den Schraubenschaft (130; 230) hält; und
einer Abdeckung (10a, 10b; 20a), die abnehmbar an der Rahmenkonstruktion (10; 20) angebracht ist.

4. Unterstützungsvorrichtung (1; 2) gemäß Anspruch 4, wobei die Rahmenkonstruktion (10; 20) aufweist:
ein unteres Stützelement (100; 201), das einen unteren Teil des Schraubenschafts (130; 230) drehbar hält;
ein oberes Stützelement (101; 200), das einen oberen Teil des Schraubenschafts (130; 230) drehbar hält; und
eine erste Säule (102; 202), die vor der Schraubenwelle (130; 230) bereitgestellt ist, um das untere Stützelement (100; 201) mit dem oberen Stützelement (101; 200) zu verbinden, und
die Unterstützungsvorrichtung (1) hat:
eine Schiene (160; 260), die entlang der ersten Säule (102; 202) bereitgestellt ist und so konfiguriert ist, dass sie die Bewegung des Linearbewegungselements (14; 24) führt; und
einen Führungsblock (161; 261), der an dem Linearbewegungselement (14; 24) befestigt ist und so konfiguriert ist, dass er entlang der Schiene (160; 260) gleitet.

5. Unterstützungsvorrichtung (1; 2) gemäß Anspruch 5, wobei
der Motor (11; 21) hinter der Schraubenwelle (130; 230) bereitgestellt ist, und
die Rahmenkonstruktion (10; 20) ein Paar linker und rechter zweiter Säulen (103, 104; 203) hat, die links und rechts von der Spindelwelle (130; 230) oder dem Motor (11; 21) bereitgestellt sind, so dass das untere Stützelement (100; 201) mit dem oberen Stützelement (101; 200) verbunden ist.

6. Unterstützungsvorrichtung (1) gemäß Anspruch 5 oder 6, wobei
die Rahmenkonstruktion (10) eine Basis (105) hat, die unter dem unteren Stützelement (100) bereitgestellt ist, um dem unteren Stützelement (100) zugewandt zu sein, und mit dem unteren Stützelement (100) verbunden ist, und
die Abdeckung (10a, 10b) an der linken und rechten Seite des oberen Stützelements (101) angebracht ist und unterhalb oder oberhalb der Basis angebracht ist.

7. Unterstützungsvorrichtung (1) gemäß einem der Ansprüche 4 bis 7, mit:
einem ersten Stützabschnitt (41), der an der Rahmenkonstruktion (10; 20) bereitgestellt ist;
einem zweiten Stützabschnitt (42), der so konfiguriert ist, dass er sich im Einklang mit dem Linearbewegungselement (14; 24) bewegt, um sich zwischen einer Position über dem ersten Stützabschnitt (41) und einer Position unter dem ersten Stützabschnitt (41) zu bewegen; und
einem dritten elastischen Element (40), dessen eines Ende drehbar von dem ersten Stützabschnitt (41) und dessen anderes Ende drehbar von dem zweiten Stützabschnitt (42) gestützt ist.

8. Künstliches Glied mit der Unterstützungsvorrichtung (1; 2) gemäß einem der Ansprüche 1 bis 8.

## Revendications

1. Dispositif d'assistance (1 ; 2) pour assister un mouvement d'une articulation (172) comprenant :
une vis à billes (13 ; 23) configurée pour convertir un mouvement de rotation d'un arbre à vis (130 ; 230) tourné par un moteur (11 ; 21) en un mouvement linéaire d'un écrou (131 ; 231) ;
un élément de mouvement linéaire (14 ; 24) disposé coaxialement avec l'arbre à vis (130 ; 230) et configuré pour se déplacer le long de l'arbre à vis (130 ; 230) avec l'écrou (131 ; 231) ;
un élément élastique (150, 151 ; 250, 251) disposé coaxialement avec l'arbre à vis (130 ; 230) de manière à s'interposer entre l'écrou (131 ; 231) et l'élément de mouvement linéaire (14 ; 24) ; et
un mécanisme de réduction de vitesse (50) configuré pour réduire une vitesse de rotation du moteur (11 ; 21) et pour transmettre une puissance du moteur (11 ; 21) à la vis à billes (13 ; 23),
**caractérisé par**
un mécanisme à manivelle (17 ; 27) configuré pour convertir le mouvement linéaire de l'élément de mouvement linéaire (14 ; 24) en un mouvement de rotation, dans lequel
le mécanisme de réduction de vitesse (50) comprend :
un petit engrenage (51) fixé à un arbre d'entraînement du moteur (11 ; 21) ; et
un grand engrenage (54) fixé à l'arbre à vis (130 ; 230), ayant un diamètre plus grand que le petit engrenage (51), et s'engrenant avec le petit engrenage (51) par l'intermédiaire d'un autre engrenage (53) ou s'engrenant directement avec le petit engrenage (51).

2. Dispositif d'assistance (1 ; 2) selon la revendication 1 ou 2, dans lequel
l'élément élastique (150, 151 ; 250, 251) comprend :
un premier élément élastique (150 ; 250) disposé au-dessous de l'écrou (131 ; 231) ; et
un deuxième élément élastique (151 ; 251) disposé au-dessus de l'écrou (131 ; 231), et
l'élément de mouvement linéaire (14 ; 24) est un cylindre qui intègre l'écrou (131 ; 231), le premier élément élastique (150 ; 250) et le deuxième élément élastique (151 ; 251).

3. Dispositif d'assistance (1 ; 2) selon l'une des revendications 1 à 3, comprenant :
un cadre (10 ; 20) qui supporte l'arbre à vis (130 ; 230) ; et
un couvercle (10a, 10b ; 20a) fixé de manière amovible au cadre (10 ; 20).

4. Dispositif d'assistance (1 ; 2) selon la revendication 4, dans lequel le cadre (10 ; 20) comprend :
un élément de support inférieur (100 ; 201) qui supporte de manière rotative une partie inférieure de l'arbre à vis (130 ; 230) ;
un élément de support supérieur (101 ; 200) qui supporte de manière rotative une partie supérieure de l'arbre à vis (130 ; 230) ; et
un premier pilier (102 ; 202) disposé devant l'arbre à vis (130
; 230) pour relier l'élément de support inférieur (100 ; 201) à l'élément de support supérieur (101 ; 200), et
le dispositif d'assistance (1) comprenant :
un rail (160 ; 260) disposé le long du premier pilier (102 ; 202) et configuré pour guider le mouvement de l'élément de mouvement linéaire (14 ; 24) ; et
un bloc de guidage (161 ; 261) qui est fixé à l'élément de mouvement linéaire (14 ; 24) et configuré pour glisser le long du rail (160 ; 260).

5. Dispositif d'assistance (1 ; 2) selon la revendication 5, dans lequel le moteur (11 ; 21) est disposé derrière l'arbre à vis (130 ; 230), et le cadre (10 ; 20) comprend une paire de seconds piliers gauche et droit (103, 104 ; 203) disposés à gauche et à droite de l'arbre à vis (130 ; 230) ou du moteur (11 ; 21) de manière à relier l'élément de support inférieur (100 ; 201) à l'élément de support supérieur (101 ; 200).

6. Dispositif d'assistance (1) selon la revendication 5 ou 6, dans lequel
le cadre (10) comprend une base (105) qui est disposée au-dessous de l'élément de support inférieur (100) pour faire face à l'élément de support inférieur (100) et qui est reliée à l'élément de support inférieur (100), et
le couvercle (10a, 10b) est attaché aux côtés gauche et droit de l'élément de support supérieur (101) et attaché au-dessous ou au-dessus de la base.

7. Dispositif d'assistance (1) selon l'une des revendications 4 à 7, comprenant :
une première partie de support (41) disposée sur le cadre (10 ; 20) ;
une deuxième partie de support (42) configurée pour se déplacer à l'unisson avec l'élément de mouvement linéaire (14 ; 24) pour se déplacer entre une position au-dessus de la première partie de support (41) et une position au-dessous de la première partie de support (41) ; et
un troisième élément élastique (40) dont une extrémité est supportée de manière rotative par la première partie de support (41) et l'autre extrémité est supportée de manière rotative par la deuxième partie de support (42).

8. Membre artificiel comprenant le dispositif d'assistance (1 ; 2) selon l'une des revendications 1 à 8.
